# EUROPEAN PATENT SPECIFICATION

(11) **EP 1 250 140 B1**
(45) Date of publication and mention of the grant of the patent: **27.05.2009**
(21) Application number: 00985244.3
(22) Date of filing: 22.12.2000
(51) Int. Cl.: A61K 31/00, A61K 31/70, A61K 45/06, A61P 35/00

(54) **COMBINATION OFAN ABL-, PDGF-RECEPTOR- AND/OR KIT RECEPTOR-TYROSINE KINASE INHIBITOR WITH AN ORGANIC COMPOUND CAPABLE OF BINDING TO ALPHA1-ACIDIC GLYCOPROTEIN**
KOMBINATION EINES ABL-, PDGF-REZEPTOR- UND/ODER KIT REZEPTOR-TYROSIN KINASE INHIBITORS MIT EINER ORGANISCHEN SUBSTANZ, DIE AN ALPHA1-ACIDISCHES GLYCOPROTEIN BINDET
COMBINAISON D'UN INHIBITEUR DE ABL-, PDGF-RECEPTEUR- ET/OU KIT RECEPTEUR-TYROSINE KINASE ET D'UN COMPOSE ORGANIQUE CAPABLE DE SE LIER A UNE GLYCOPROTEINE ALPHA1-ACIDE

(30) Priority: 27.12.1999 IT MI992711
(43) Date of publication of application: 23.10.2002
(73) Proprietor: NOVARTIS AG, 4056 Basel (CH); Novartis Pharma GmbH, 1230 Wien (AT)
(72) Inventor: GAMBACORTI-PASSERINI, Carlo, I-20052 Monza (IT); LECOUTRE, Philipp, 10117 Berlin (DE)
(74) Representative: Gros, Florent
(86) International application number: PCT/EP2000/013161
(87) International publication number: WO 2001/047507

(56) References cited:
- WO-A-01/04125
- WO-A-01/42246
- WO-A-99/03854
- TOPALY J ET AL: "SYNTERGISTIC ACTIVITY OF THE NEW ABL-SPECIFIC TYROSINE KINASE INHIBITOR ST1571 AND CHEMOTHERAPEUTIC DRUGS ON BCR-ABL-POSITIVE CHRONIC MYELOGENOUS LEUKEMIA CELLS" LEUKEMIA, MACMILLAN PRESS LTD, US, vol. 15, May 2001 (2001-05), pages 342-347, XP001039741 ISSN: 0887-6924
- GAMBACORTI-PASSERINI C. ET AL: "Role of alpha-1 acid glycoprotein in the in vivo resistance of human BCR-ABL+ leukemic cellst to the ABL inhibitor STI571" J NATL. CANCER INST., vol. 92, no. 20, 18 October 2000 (2000-10-18), pages 1641-1650, XP001036600
- WEISBERG E. ET AL: "Mechanisms of resistance imatinib (STI571) in preclinical models and in leukemia patients" DRUG RESISTANCE UPDATES, vol. 4, 2001, pages 22-28, XP001039532

## Description

This invention relates to combinations of an abl-, PDGF-Receptor- and/or Kit receptor-tyrosine kinase inhibitor with an organic compound capable of binding to α₁-acidic glycoprotein (AGP), as well as to pharmaceutical preparations, in relation to disease states which respond to inhibition of abl-, PDGF-Receptor- and/or Kit-receptor tyrosine kinase. In particular, the invention relates to products or combinations comprising an abl-, PDGF-Receptor- and/or Kit receptor-tyrosine kinase inhibitor with an organic compound capable of binding to α₁-acidic glycoprotein, either in fixed combination or for chronologically staggered or simultaneous administration, and the combined use of both classes of compounds, either in fixed combination or for chronologically staggered or simultaneous administration for the manufacture of a medicament for the treatment of proliferative diseases, especially tumor diseases, especially those that can be treated by inhibition of abl-, PDGF-Receptor- and/or Kit receptor-tyrosine kinase activity.

### Background of the Invention

A number of compounds are known to inhibit the proliferation of cells by way of inhibition of either the abl-, the PDGF-Receptor and/or the Kit receptor tyrosine kinase. For example, International Application No. WO 97/02266, International Patent Application WO98/35958 and especially European Patent Application EP 0 564 409-A, as well as International Application No. WO99/03854 mention compounds that are inhibitors of at least one of the tyrosine kinases mentioned above. Further compounds that are of interest are Tyrphostin AG957 (see Kaur et al., Anticancer Drugs 5, 213-222 (1994), Herbimycin A (Okabe and Uehara, Leukemia and Lymphoma 12, 2156-2162 (1994), Blood 80, 1330-1338 (1994) and Leuk. Res. 18, 213-220 (1994), as well as Rioran et al., Oncogene 16, 133-1542 (1998)); Tyrphostins AG 1295, AG 1296 (see Kovalenko et al., Cancer Res. 54, 6106-6114 (1994), Lipson et al., Pharmacol & Exp- Therap. 285, 844-852 (1998), Krystal et al., Cancer Res. 57, 2203-2208 (1997)); SU 101 (Leflunomide), as well as its metabolite (see Shawer et al., Clin. Cancer Res. 3, 1167-1177 (1997), Mattar et al., FEBS Lett. 334, 161-164 (1993), Cherwinskyi et al., Inflamm. Res. 3, 1167-1177 (1997), and Strawn et al., Exp. Opin. Invest. Drugs 7, 533-573 (1998)); and Pyridopyrimidines (see e.g. Hamby et al., J. Med. Chem. 40, 2296-2303 (1997), Dahring et al., J. Pharmacol. Exp. Ther. 281, 1446-1456 (1997), Klutcho et al., Life Sci. 62,143-150 (1998), Panek et al., J. Pharmacol. Exp. Ther. 283, 1433-1444 (1997), Boschelli et al., J. Med. Chem. 41, 4365-4377 (1998)).

These compounds, as described in the mentioned patent applications and other publications, have been shown to be effective in the prophylaxis and especially treatment of diseases that are caused by deregulation of the phosphorylation and/or activity of the tyrosine kinases just mentioned.

The phosphorylation of proteins has long been known as an essential step in the differentiation and division of cells. Phosphorylation is catalysed by protein kinases subdivided into serine/threonine and tyrosine kinases. The tyrosine kinases comprise PDGF (Platelet-Derived Growth Factor) receptor tyrosine kinase = PDGF-R TK, abl tyrosine kinase (abl), and kit receptor tyrosine kinase (kit R TK). Where these tyrosine kinases are deregulated, e.g. by way of mutation or activation through external factors, e.g. compounds (internal natural compounds, such as PDGF, or external compounds) binding to them, *inter alia* deregulation of cell growth is the result.

PDGF (platelet-derived growth factor) is a very frequently occurring growth factor which plays an important role both in normal growth and in pathological cell proliferation, such as in carcinogenesis and disorders of the smooth muscle cells of blood vessels, for example in atherosclerosis and thrombosis. Its inhibition can be measured in analogy to the procedure described in EP 0 564 409 mentioned above (see also E. Andrejauskas-Buchdunger and U. Regenass in Cancer Research 52, 5353-5358 (1992)).

The inhibition of abl kinase, e.g. v-abl-tyrosine kinase is determined in accordance with the methods of N. Lydon et al., Oncogene Research 5_, 161-173 (1990) and J. F. Geissler et al., Cancer Research 52, 4492-4498 (1992). In those methods [Val⁵]-angiotensin II and [γ³²P]-ATP are used as substrates.

The inhibition of kit receptor tyrosine kinase can be measured e.g. as *in vitro* c-Kit kinase assay:

The *in vitro* c-Kit kinase assay is performed in 96-well plates as a filter binding assay, using the recombinant GST(glutathione S transferase)-fused c-Kit kinase domain expressed in baculovirus and purified over glutathione-Sepharose. The GST-fusion protein is incubated under optimized conditions in the presence or absence of drug and kinase inhibition is measured by dectecting the decrease in phosphorylation of the poly(GluTyr)(4:1) peptide P-275. Gamma-[³³P]-ATP is used as the phosphate donor. Atematively, it is possible to use a cellular assay for c-Kit: C-Kit overexpressing cells are serum-starved and incubated for 90 min at 37°C with the drug prior to stimulation with recombinant human stem cell factor. Equal amounts of protein from cell lysates are analyzed for inhibition of c-Kit phosphorylation by Western blotting using anti-phosphotyrosine antibodies.

Owing to the properties described, compounds that show inhibition of one of the tyrosine kinases mentioned above can be used as therapeutics, especially for the treatment of proliferative diseases, such as cancer, especially tumors and leukemias.

The compounds, on the other hand, can be used not only as tumor-inhibiting active ingredients but also as drugs against non-malignant proliferative diseases, e.g. atherosclerosis, thrombosis, psoriasis, sclerodermitis and fibrosis. They are also suitable for the further applications mentioned above for protein kinase C-modulators and can be used especially in the treatment of diseases that respond to the inhibition of PDGF-receptor kinase.

A tyrosine kinase inhibitor as described above also inhibits BCR/Abi kinase (see Nature Medicine 2, 561-566 (1996)) and is thus suitable for the treatment of BCR/Abl-positive cancer and tumor diseases, such as leukemias (especially chronic myeloid leukemia and acute lymphoblastic leukemia, where especially apoptotic mechanisms of action are found), and also shows effects on the subgroup of leukemic stem cells as well as potential for the purification of these cells *in vitro* after removal of said cells (for example, bone marrow removal) and reimplantation of the cells once they have been cleared of cancer cells (for example, reimplantation of purified bone marrow cells).

In addition, a tyrosine kinase inhibitor as described above can show useful effects in the treatment of disorders arising as a result of transplantation, for example, allogenic transplantation, especially tissue rejection, such as especially obliterative bronchiolitis (OB), i.e. a chronic rejection of allogenic lung transplants. In contrast to patients without OB, those with OB often show an elevated PDGF concentration in bronchoalveolar lavage fluids. The tyrosine kinase inhibtors can also be effective in diseases associated with vascular smooth-muscle cell migration and proliferation (where PDGF and PDGF-R often also play a role), such as restenosis and atherosclerosis. They may also be able of inhibiting angiogenesis.

All these uses are described in detail in EP 0 564 409, WO 97/02266, WO 99/03854 and WO 98/35958, or the other above-mentioned references.

One example of a compound that shows inhibitory activity on the above-mentioned tyrosine kinases is the compound named 4-(4-methylpiperazin-1-ylmethyl)-N-[4-methyl-3-(4-pyridin-3-yl)pyrimidin-2-ylamino)phenyl]benzamide, which is described in EP 0 564 409 and, in the form of the methane sulfonate salt (STI571 hereinafter), preferably in the β-crystal form, in WO 99/03854.

This compound is a potent inhibitor of bcr/abl, an oncogenic fusion protein that causes Chronic Myeloid Leukemia (CML). However, it has been observed in an ongoing clinical trial that CML patients in blast crisis and relapsed Philadelphia Chromosome Positive Acute Lymphoblastic Leukemia (Ph+-ALL) patients show only temporary responses to STI571, which are followed in a brief period of time by the development of resistance.

We previously showed that this compound can cure mice injected with human BCR/ABL+ leukemic cells, if continuous inhibition of the kinase activity of bcr/abl is maintained. This model was used to study the possible development of resistance to STI571. When animals bearing large tumors (>400 mg) were treated, tumor reduction was observed in all animals, with disappearance in some. However, no animal was cured, relapsed animals did not respond to further treatment, and the bcr/abl kinase activity was not inhibited by STI571 administration *in vivo,* although active plasma concentrations (≈10 µM) were obtained. Tumors were excised from relapsed, resistant animals, placed in culture, and tested within 24 hours for *in vitro* sensitivity to Stet571. 5/5 tumors examined showed IC₅₀ (0.1-0.3 µM) not significantly different from that of the parental KU612 line.

Resistance can develop as the result of several factors, operating either at cellular level or only *in vivo.* Drug resistance can develop, for example, as a result of mutation/amplification of the target gene, induction of metabolism, and, paradoxically, increased post-translational degradation of the target protein, and the like.

Surprisingly, it has been found that pharmacokinetics data indicated that relapsed animals reached similar peak concentrations as controls, but they showed a significantly slower decrease in ST1571 concentrations over time. We have now found that this is due to the presence of a binding factor in the plasma of relapsed animals, able to decrease tissue distribution and clearing of STI571. A number of proteins were tested *in vitro* for their ability to inhibit the biological activity of STI571. While albumin does not influence STI571 inhibition of KU812 proliferation, α₁-acidic glycoprotein (AGP) does at physiological concentrations, increasing the IC₅₀ for STI571 up to 90 fold. AGP also inhibits the effect of STI571 on bcr/abl phosphorylation *in vitro.* Association Constant (Ka) for specific binding to STI571 is calculated and found to be 60 times higher in AGP than in albumin. AGP levels are measured in mice by an immunoassay. A strong correlation is found between tumor load and AGP concentrations. In addition, pretreatment with STI571 *in vivo* also increases AGP plasma levels. Accordingly, animals pretreated with STI571, and then injected with KU812 cells and treated with STI571 24 hours later, are less responsive to treatment than controls (cured animals: 8/16 vs. 16/16, p<0.01). These results suggest that rising AGP levels, either induced from the tumor or from the treatment itself, are responsible for the development of resistance to STI571.

The present invention has the task to provide a means to overcome the resistance that develops in warm-blooded animals if STI571 or any other of the tyrosine kinase inhibitors mentioned above is applied during treatment of a disease as mentioned above.

The surprising result that the resistance against a tyrosine kinase inhibitor, especially STI571, may be due to AGP binding and thus lower free concentration of the active drug in blood plasma forms a basis for the search for a solution, be it by way of stopping a regulatory molecule, e.g. a regulatory protein, from allowing the activation of the transcription of the AGP gene, by way of stopping amplification of the AGP gene, by way of inhibition of genetic transcription of the AGP coding mRNA, by way of inhibition of translation of said mRNA into the mature protein, by way of influencing its distribution and finishing to the final glycoprotein, by way of inhibiting its secretion into blood plasma, by way of larger dosing of the tyrosine kinase inhibitor, by way of neutralizing AGP e.g. with antibodies, by way of activating metabolism or elimination from plasma, by way of activating post-translational degradation of AGP or its precursors, and the like.

Unexpectedly, in view of reports that stated that there is doubt on the relevance of drug displacement during combined drug therapy (see e.g. Kremer et al., Pharmacological Rev. 40 (1), 1-47 (1988)), it has now been found that it is possible, by combining one or more AGP binding compounds with an abl-, PDGF-Receptor- and/or Kit receptor-tyrosine kinase inhibitor, to overcome this type of resistance.

Therefore, the present invention allows for an important improvement in therapy of patients that have one of the diseases mentioned in the present disclosure.

### Summary of the invention

This invention relates to a combination of (a) an abl-, PDGF-Receptor- and/or Kit receptor-tyrosine kinase inhibitor (component (a)) and (b) an organic compound capable of binding to α₁-acidic glycoprotein (AGP) (component (b)), as well as to pharmaceutical preparations, in relation to disease states which respond to inhibition of abl-, PDGF-Receptor- and/or Kit-receptor tyrosine kinase. In particular, the invention relates to products or combinations comprising (a) an abl-, PDGF-Receptor- and/or Kit receptor-tyrosine kinase inhibitor and (b) an organic compound capable of binding to α₁-acidic glycoprotein (AGP), either in fixed combination or for chronologically staggered or simultaneous administration, and the combined use of both classes of compounds, either in fixed combination or for chronologically staggered or simultaneous administration for the manufacture of a medicament for the treatment of proliferative diseases, especially tumor diseases, especially those that can be treated by inhibition of abl-, PDGF-Receptor- and/or Kit receptor-tyrosine kinase activity.

### Description of the Figures

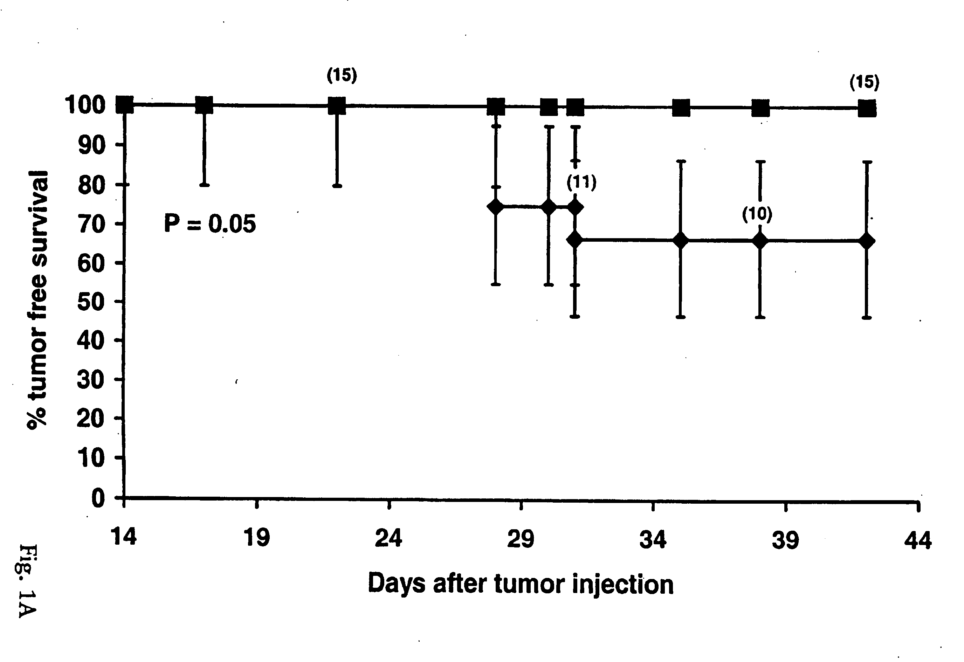

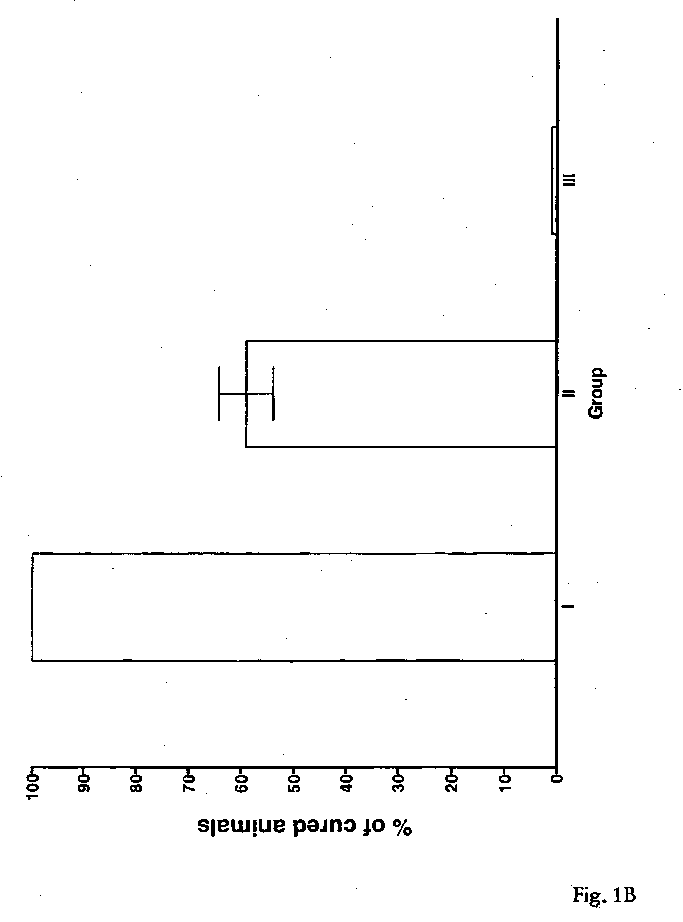

Fig. 1A and B shows the effects of initial tumor load and of length of STI571 treatment.
   Fig. 1 A: Two groups of 15 nude mice are injected with 50 x 10⁶ KU812 leukemic cells. Treatment with STI571 (160 mg/kg p.o. every 8 hours for 11 days) is started after 1 day (group I; squares) or after 8 days (group II; diamonds) in the presence of a mean tumor weight of 276±97 mg. The numbers in parentheses indicate the number of tumor-free animals.
   Fig. 1 B: Nude mice injected with KU812 cells are treated with STI571 (160 mg/kg p.o. every 8 hours for 11 days) after 1 day (group I), after 8 days (group II, mean tumor weight 253±122 mg), or after 15 days (group III, mean tumor weight 1054±258 mg). The results represent the mean of three consecutive experiments.
   Fig. 1C: Animals belonging to group II are left untreated (controls) or treated with STI571 (160 mg/kg p.o. every 8 hours) for 11 or 18 days with STI571.
Fig. 2 shows the effect of re-treatment with STI571 on tumor relapsing after an initial response to STI571. Dashed lines refer to the growth if untreated tumors (see methods part in the examples).
Fig. 3 shows the *in vitro* sensitivity to STI571 of two *in vivo* resistant tumors. Values are expressed as % of controls which incorporated 129'362±6329 cpm.
Fig. 4 shows the *in vivo* inhibition of Bcr/Abl kinase activity by STI571. Tumor-bearing mice are acutely treated with STI571 orally (160 mg/kg) and killed at various time points. Tumor samples are extracted and used for western blot analysis with anti-phosphotyrosine (pTyr) or anti-Abelson (Abl). STI571 efficiently inhibits phosphorylation of the BCR/ABL tyrosine kinase in controls (Ctrl) at 2 and 4 hours (80% and 50% inhibition by densitometric analysis) compared to non-treated animals (n.t.), while extracts from relapsed animals (Rel) are resistant to STI571 treatment.
Fig. 5 shows plasma and tumor concentrations of STI571 in tumor bearing mice pretreated or not with STI571. Animals are acutely treated with STI571 (160 mg/kg p.o.) and killed 0.5, 2 and 5 hours later. STI571 is determined by HPLC in plasma samples and in tumor extracts. Control mice never receive a previous treatment with STI571, while pretreated mice are subjected to two 11 days cycles of STI571 (as in Fig. 2). Average tumor weights are 450±129 mg in controls and 684±283 mg in pretreated mice.
Fig. 6 shows the *in vitro* sensitivity to STI571 of KU812 cells in the presence of α₁-acidic glycoprotein (A) and Albumin (B) (see ³H-thymidine uptake under methods).
Fig. 7 shows the effect of two serum samples containing different amounts of AGP on the *in vitro* activity of STI571 on KU812 cells.
Fig. 8A and B show the determination of AGP in normal and tumor bearing mice.

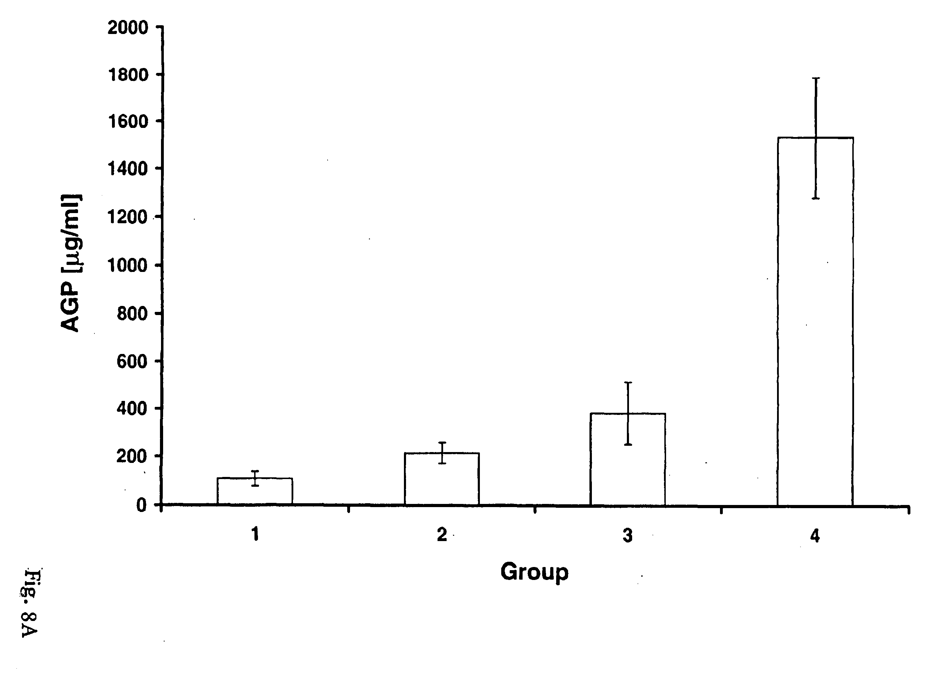

   Fig. 8A: Average AGP plasma concentrations in mice with different disease or treatment status. Group 1 (n=11) refers to normal mice, group 2 (n=8) to mice treated with STI571 for 11 days (and sampled 3 days after treatment discontinuation), group 3 (n=6) to mice bearing an 8 day old tumor (average weight 304±116 mg), group 4 (n=7) to mice bearing a 15 days old tumor (average weight 1184±295 mg).

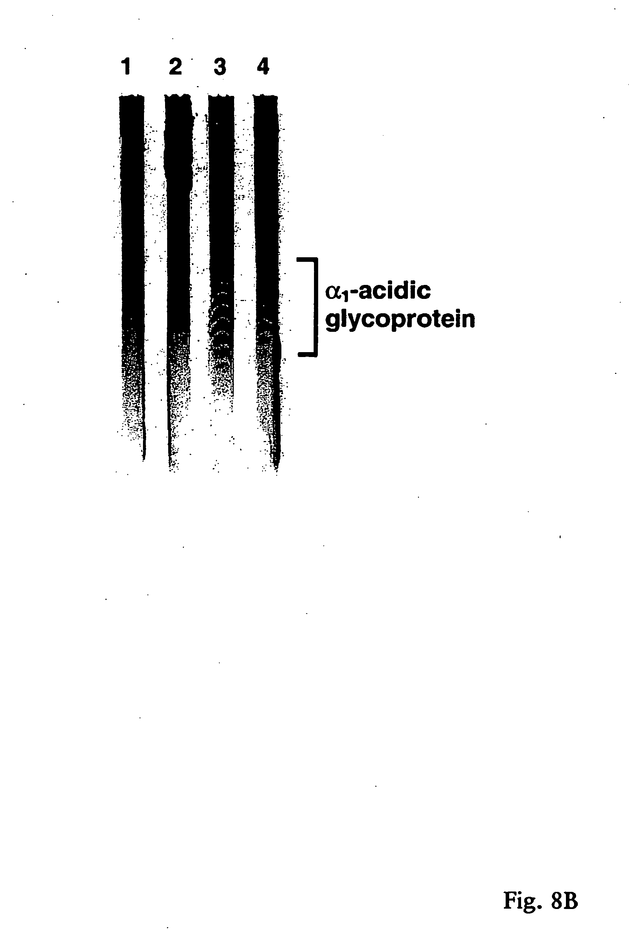

   Fig. 8B: Direct determination of AGP in normal and tumor-bearing mice by isoelectrofocusing: lanes 1-2 refer to normal mice, lanes 3-4 to animals bearing large (>1 g) tumors. The different AGP isoforms are indicated and are comprised between pH 3.4 and 4.0.

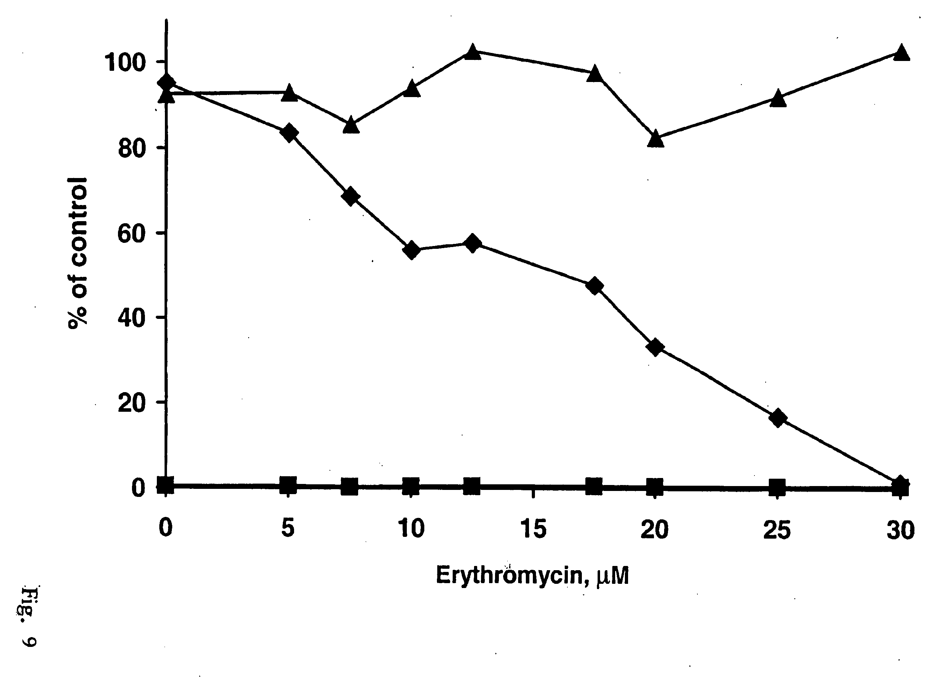

Fig. 9 shows the effect of AGP (at 1 mg/ml) and erythromycin on the activity of STI571 (at 1 µM) on KU812 cells.

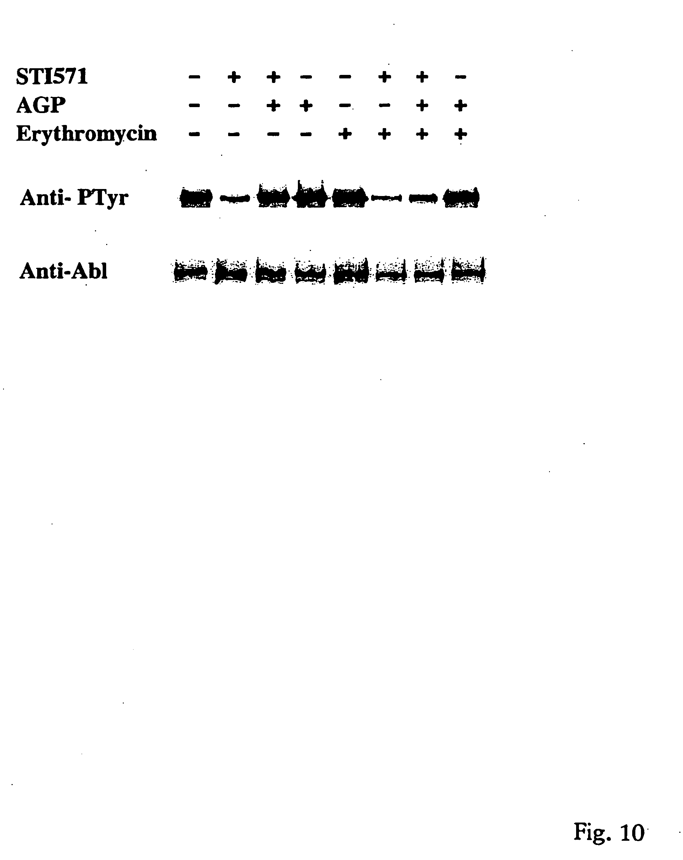

Fig. 10 shows the effect of AGP and erythromycin on the inhibition of bcr/abl autiphosphorylation induced by STI571.
   3 x 10⁶ cells per well are incubated at 37 °C with eryrthromycin base (100 µM), STI571 (3 µM), AGP (2mg/ml). After 1 hour, cells are washed twice with cold phosphate-buffered saline (PBS) and subsequently lysed in 500 µl of 1 x Laemmli's buffer. Cell lysates corresponding to 90,000 cells are analysed by SDS Electrophoresis on 7.5% polyacrylamide. Endogenous bcr/abl and tyrosine-phosphorylated bcr/abl are detected with the corresponding mouse monoclonal antibody.

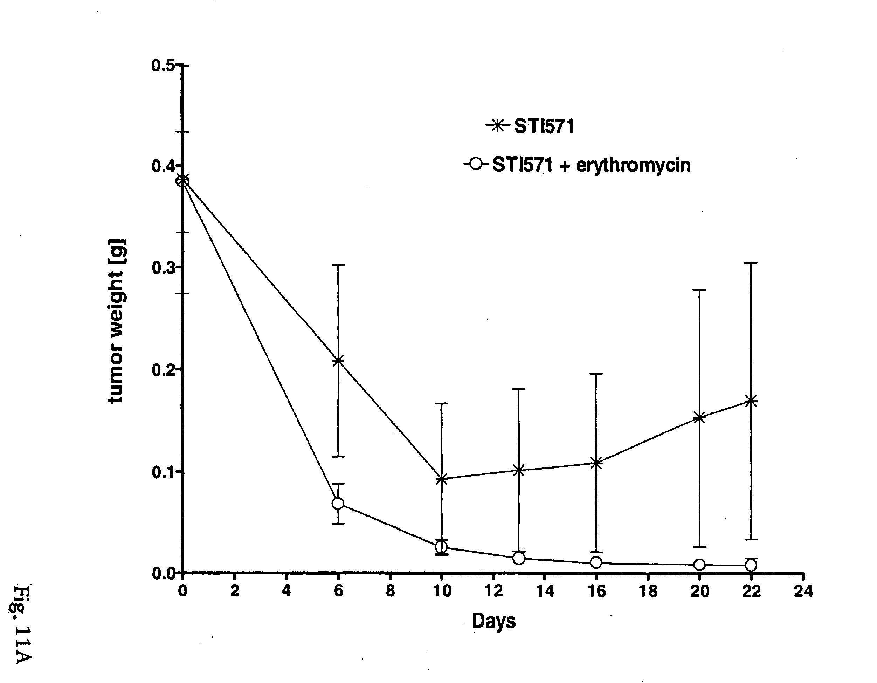

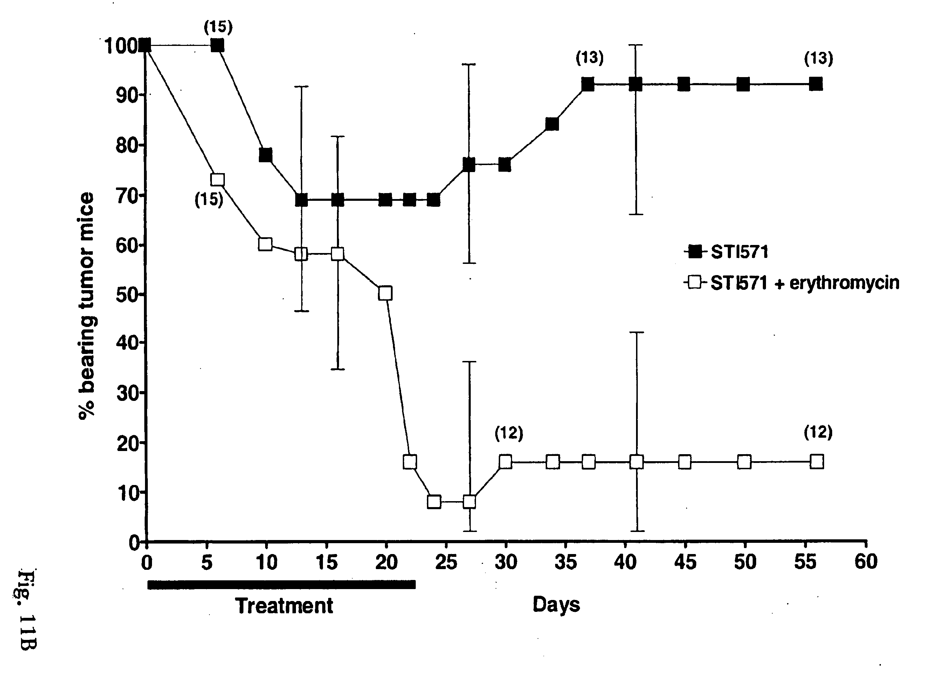

Fig. 11A and B show the *in vivo* effect of erythromycin on co-administration with STI571 to tumor bearing mice. Animals bearing 11 days old tumors are randomly assigned to two separate groups. 15 mice are treated with STI571 and erythromycin (average tumor weight 385±53 mg), whereas another 15 mice received STI571 only (mean tumor weight 390±114 mg). Controls groups receive erythromycin alone resuspended in methyl cellulose 5% (5 mice) or methyl cellulose 5% only (6 mice).
   Fig. 11A: Mean tumor weights during treatment (day 0-20).
   Fig. 11 B: Percent of tumor bearing mice: at each time point the percentage of mice bearing a palpable tumor is calculated on the total number of mice alive at that moment. The number of mice alive at a certain time point is indicated by the numbers in parentheses (during the experiment 2 and 3 animals were killed accidentally in the group receiving STI571 only and in the group receiving the combined treatment, respectively).

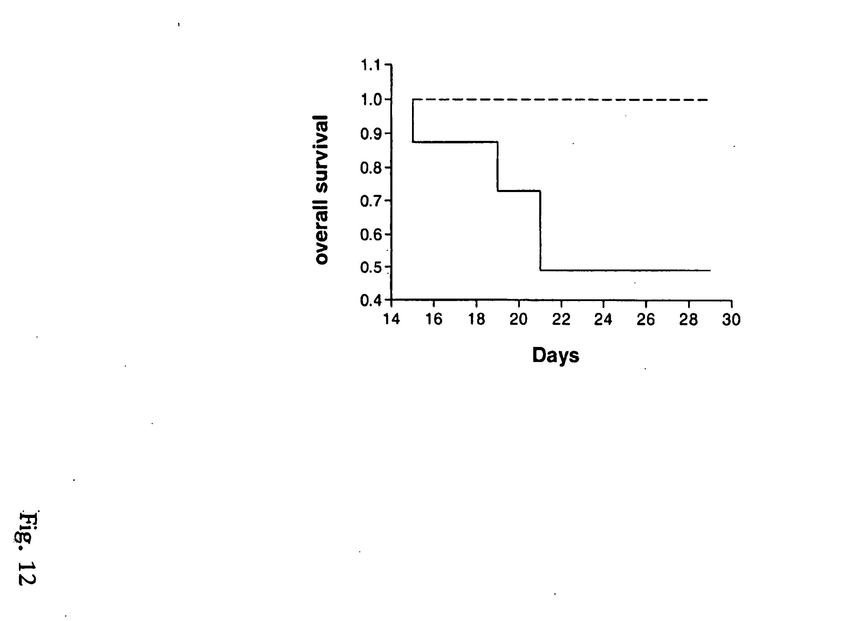

Fig. 12 shows the effect of pretreatment on the anti-leukemic effect of STI571. Two groups of nude mice are treated with STI571 (160 mg/kg every 8 hours) for 11 days, starting 1 day after leukemic cell injection. The dashed line refers to control, non-pretreated animals. The solid line refers to animals that have received an identical STI571 treatment 14 days before being injected with KU812 bcr/abl+ leukemic cells.

### Detailed Description of the invention

Surprisingly, positive and preferably even synergistic effects between abl-, PDGF-R- and or Kit receptor tyrosine kinase inhibitors and organic compounds capable of binding to α₁-acidic glycoprotein (AGP) have been observed in nude mouse xenograft models. This is evidence that the tyrosine kinase inhibitors may be used not only as single agents, but also especially in combination therapy with organic compounds capable of binding to AGP for the treatment of cancer diseases.

This combination offers a lot of advantages: In the first place, tyrosine kinase inhibitors may display significant side effects up to really toxic effects, so that simply compensating AGP binding by an increase of the dose of a tyrosine kinase inhibitor is often very difficult or impossible in order to obtain a responsible balance between therapeutic use and side effects. In the new combinations described herein, however, it is possible to diminish the amount of tyrosine kinase inhibitor needed and thus to alleviate side effects. Second, the organic compound that may be used as compound capable of binding to AGP may be selected from compounds with very high tolerability, thus allowing great flexibility in the treatment of cancer patients. Third, due to the fact that the release of pharmaceutically active tyrosine kinase inhibitors from AGP binding in plasma opens up a totally new route of treatment, it is also possible to treat cancer types which have been very difficult to treat or even practically unaffected by therapy with standard chemotherapeutics. Most importantly, it is possible to overcome *in vivo* desensibilisation (resistance) of proliferating cells to a tyrosine kinase inhibitor, which may be present either already at the beginning of the treatment (e.g. preferably due to high AGP levels in blood plasma) or may have developed or is developing during treatment with an abl-, PDGF-R- and/or Kit receptor-tyrosine kinase inhibitor as described in the present disclosure. The present invention preferably relates to the use of a combination of (a) at least one abl-, PDGF-R- and/or Kit receptor-tyrosine kinase inhibitor and (b) at least one organic compound capable of binding to α₁-acidic glycoprotein (AGP); or pharmaceutically acceptable salts of any component (a), (b) or (a) and (b) if at least one salt-forming group is present, for producing a pharmaceutical preparation for use as compositions against a proliferative disease that can be treated by administration of an abl-, PDGF-R- and/or Kit receptor-tyrosine kinase inhibitor, which is or, during treatment with 4-(4-methylpiperazin-1-ylmethyl)-N-[4-methyl-3-(4-pyridin-3-yl)pyrimidin-2-ylamino)phenyl]benzamide, is becoming or has become completely or partially resistant to such treatment.

The invention also relates to a product which comprises
(a) at least one abl-, PDGF-R- and/or Kit receptor-tyrosine kinase inhibitor and
(b) at least one organic compound capable of binding to α₁-acidic glycoprotein (AGP),
wherein any component (a) and/or (b) can also be present in the form of a pharmaceutically acceptable salt, if at least one salt-forming group is present,

in the presence or absence of one or more pharmaceutically acceptable carrier materials, as a combination preparation for simultaneous or chronologically staggered use within a period of time which is small enough for the active compounds both of component (a) and of component (b) to enhance antiproliferative activity of compound (a) against proliferating cells, especially in a patient, for treating a proliferative disease which responds to such a compound, wherein the abl-, PDGF-Receptor- and/or Kit receptor-tyrosine kinase inhibitor is selected from the group consisting of
(i) 1-(4-chloro-anilino)-4-(4-pyridyl-methyl)-phthalazine and
(ii) an N-phenyl-2-pyrimidine-amine derivative of formula **I** 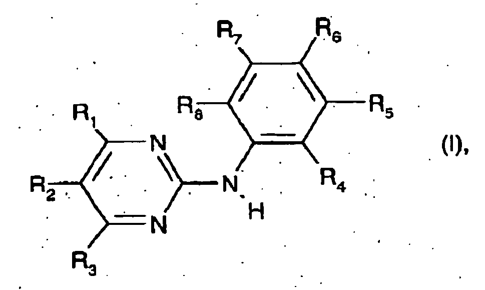 wherein
   R₁ is pyrazinyl,1-methyl-1H-pyrrolyl, amino- or amino-lower alkyl-substituted phenyl wherein the amino group in each case is free, alkylated or acylated, 1H-indolyl or 1H-imidazolyl bonded at a five-membered ring carbon atom, or unsubstituted or lower alkyl-substituted pyridyl-bonded at a ring carbon atom and unsubstituted or substituted at the nitrogen atom by oxygen,
   R₂ and R₃ are each independently of the other hydrogen or lower alkyl,
   one or two of the radicals R₄, R₅, R₆, R₇ and R₈ are each nitro, fluoro-substituted lower alkoxy or a radical of formula II

   -N(R₉)-C(=X)-(Y)ₙ-R₁₀ (II)

   wherein
   - R₉: is hydrogen or lower alkyl,
   - X: is oxo, thio, imino, N-lower alkyl-imino, hydroximino or O-lower alkyl-hydroximino,
   - Y: is oxygen or the group NH,
   - n: is 0 or 1 and
   - R₁₀: is an aliphatic radical having at least 5 carbon atoms, or an aromatic, aromatic-aliphatic, cycloaliphatic, cycloatiphatic-aliphatic heterocyclic or heterocyclic-aliphatic radical, and the remaining radicals R₄, R₅, R₆, R₇ and R₈ are each independently of the others hydrogen, lower alkyl that is unsubstituted or substituted by free or alkylated amino, piperazinyl, piperidinyl, pyrrolidinyl or by morpholinyl, or lower alkanoyl, trifluoromethyl, free, etherified or esterifed hydroxy, free, alkylated or acylated amino or free or esterified carboxy; or a salt of such compounds having at least one salt-forming group;
   and the organic compound capable of binding to α₁-acidic glycoprotein (AGP) (b) is selected from the group consisting of:
   Nicergoline, Prazosin, Alfentanil, Ketamine, Ethidocaine, Fentanil, Meperidine, Methadone, Phenylbutazone, Bupivacaine, Etidocaine, Phencyclidine, Lidocaine, Phencyclidin, Aprindine, Disopyramide, Quinidine, Verapamil, Erythromycin, Acenocoumarol, Dipyridamole, Ticlopidine, Warfarin, Phenytoin, Carbamazepine, Naproxen, Alprenolol, Metoprolol, Oxprenolol, Pindolol, Propranolol. Timolol. Progesterone. Cortexone, Testosteron, Estradiol, Prednisolone, Metocurine, d-Tubocurarine, Amitriptyline, Chlorpromazine, Cyclazindol, Desmethylimipramine; Diazepam, Doxepine, Flurazepam, Fluphenazine, Haloperidol, Imipramine, Loxapine, Mianserin, Nortriptyline, Norzimelidine, Perazine, Perphenazine, Phenobarbital, Phenothiazine derivatives, Promazine, Acepromazine, Protipendyl, Thioridazine, Thiothixene, Triazolam, Trifluoperazine, Zimelidine, Vitamin B₁₂, folic acid, 1,8-Anilino-naphthalene sulfonate, Aminopyrine, Amoxapine, Bupropion, Maprolitine, Nornifensine, Trazodone, Ritodrine, Doxazosin, Trimazosin, Binedalin, Amsacrine, Apazone, Ciclazindol, Indomethacin, Probenecid, Retinoic Acid, Sulfinpyrazone, Tolmetin, Benoxaprofen, Heparin, Sufentanil, Lofentanil, Metoclopramide, Nicardipine, Pirmenol, mifepristone, Aprindil, Auramine O, Bepridil, Desipramine, Desmethylclomipraine; Moxaprindine, Quinine, Lorcainide, Prothipendyl_{.}, Protriptyline, Trihexyphenidyl, Biperiden, Methaqualone, Diphenhydramine, Glutethimide, Chlordiazapoxid, L-Tryptophane, Mepivacaine, Levomethadone, Opipramol, Trifluopromazine, Trimipramine, tris-butoxyethyl phosphate, staurosporine, N-benzoyl-staurosporine and 7-hydroxy staurosporine;
wherein any component (a) and/or (b) can also be present in the form of a pharmaceutically acceptable salt, if at least one salt-forming group is present..

The invention also relates to a pharmaceutical preparation which comprises a quantity, which is jointly effective for treating a proliferative disease that can be treated by administration of an abi-, PDGF-R- and/or Kit receptor-tyrosine kinase inhibitor, of
(a) at least one abl-, PDGF-R- and/or Kit receptor-tyrosine kinase inhibitor and
(b) at least one organic compound capable of binding to α₁-acidic glyccprotein,
wherein any component (a) and/or (b) can also be present in the form of a pharmaceutically acceptable salt, if at least one salt-forming group is present,
with one or more pharmaceutically acceptable carrier materials,
wherein the abl-, PDGF-Receptor- and/or Kit receptor-tyrosine kinase inhibitor (a) is selected from the group consisting of
(i) 1-(4-chloro-anilino)-4-(4-pyridyl-methyl)-phthalazine
   and
(ii) an N-phenyl-2-pyrimidine-amine derivative of formula I 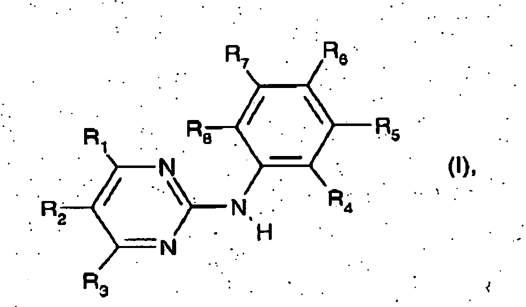 wherein
   R₁ is pyrazinyl, 1-methyl-1H-pyrrolyl, amino-or amino-lower alkyl-substituted phenyl wherein the amino group in each case is free, alkylated or acylated, 1H-indolyl or 1H-imidazolyl bonded at a five-membered ring carbon atom, or unsubstituted or lower alkyl-substituted pyridyl bonded at a ring carbon, atom and unsubstituted or substituted at the nitrogen atom by oxygen,
   R₂ and R₃ are each independently of the other hydrogen or lower alkyl,
   one or two of the radicals R₄, R₅, R₆, R₇ and R₈ are each nitro, fluoro-substituted lower alkoxy or a radical of formula II

   -N(R₉)-C(=X)-(Y)ₙ-R₁₀ (II),

   wherein
   - R₉: is hydrogen or lower alkyl,
   - X: is oxo, thio, imino, N-lower alkyl-imino, hydroximino or O-lower alkyl-hydroximino,
   - Y: is oxygen or the group NH,
   - n: is 0 or 1 and
   - R₁₀: is an aliphatic radical having at least 5 carbon atoms, or an aromatic, aromatic-aliphatic, cycloaliphatic, cycloaliphatic-aliphatic, heterocyclic or heterocyclic-aliphatic radical; and the remaining radicals R₄, R₅, R₆, R₇ and R₈ are each independently of the others hydrogen, lower alkyl that is unsubstituted or substituted by free or alkylated amino, piperazinyl, piperidinyl, pyrrolidinyl or by morpholinyl, or lower alkanoyl, trifluoromethyl, free, etherified or esterifed hydroxy, free, alkylated or acylated amino or free or esterified carboxy, or
   a salt of such compounds having at least one salt-forming group;
   and the organic compounds capable of binding to α₁-acidic glycoprotein (AGP) (b) is selected from the group consisting of:
   Nicergoline, Prazosin, Alfentanil, Ketamine, Ethidocaine, Fentanil, Meperidine, Methadone, Phenylbutazone, Bupivacaine, Etidocaine, Phencyclidine, Lidocaine, Phencyclidin, Aprindine, Disopyramide, Quinidine, Verapamil, Erythromycin, Acenocoumarol, Dipyridamole, Ticlopidine, Warfarin, Phenytoin, Carbamazopine, Naproxen, Alprenolol, Metoprolol, Oxprenolol, Pindolol, Propranolol, Timolol, Progesterone, Cortexone, Testosteron, Estradiol, Prednisolone, Metocurine, d-Tubocurarine, Amitriptyline, Chlorpromazine, Cyclazindol, Desmethylimipramine, Diazepam, Doxepine, Flurazepam, Fluphenazine, Haloperidol, Imipramine, Loxapine, Mianserin, Nortriptyline, Norzimelidine, Perazine, Perphenazine, Phenobarbital, Phenothiazine derivatives, Promazine, Acepromazine, Protipendyl, Thioridazine, Thiothixene, Triazolam, Trifluoperazine, Zimelidine, Vitamin B₁₂, folic acid, 1,8-Anilino-naphthalene sulfonate, Aminopyrine, Amoxapine, Bupropion, Maprolitine, Nomifensine, Trazodone, Ritodrine, Doxazosin, Trimazosin, Binedalin, Amsacrine, Apazone, Ciclazindol, Indomethacin, Probenecid, Retinoic. Acid, Sulfinpyrazone, Tolmetin, Benoxaprofen, Heparin, Sufentanil, Lofentanil, Metoclopramide, Nicardipine, Pirmenol, mifepristone, Aprindil, Auramine. O, Bepridil, Desipramine, Desmethylomipraine, Moxaprindine, Quinine, Lorcainide, Prothipendyl, Protriptyline, Trihexyphenidyl, Biperiden, Methaqualone, Diphenhydramine, Glutethimide, Chlordiazepoxid, L-Tryptophane, Mepivacaine, Levomethadone, Opipramol, Trifluopromazine, Trimipramine, tris-butoxyethyl phosphate, staurosporine, N-benzoyl-staurasporine and 7-hydroxy staurosporine;
   wherein any component (a) and/or (b) can also be present in the form of a pharmaceutically acceptable salt, if at least one salt-forming group is present.

The general terms used hereinbefore and hereinafter preferably have the following meanings, if not indicated otherwise:

The term "at least one" taking reference to a) abl-, PDGF-R- and/or Kit receptor-tyrosine kinase inhibitor or b) organic compounds capable of binding to α₁-acidic glycoprotein refers to one or more, especially 1 to 5, members of each group a) or b), preferably to one compound of group a) and 1 or more, especially 1 to 5, most especially 1 or 2 compounds of group b).

By the term "abl-, PDGF-R- and/or Kit receptor-tyrosine kinase inhibitor" preferably one of the following compounds is meant:

A compound mentioned in International Application No. WO 97/02266, International Patent Application WO98/3595 and especially European Patent Application EP 0 564 409-A, as well as International Application No. WO99/03854, Tyrphostin AG957 (see Kaur et al., Anticancer Drugs 5, 213-222 (1994); Herbimycin A (Okabe and Uehara, Leukemia and Lymphoma 12, 2156-2162 (1994), Blood 80, 1330-1338 (1994) and Leuk. Res. 18, 213-220 (1994), as well as Rioran et al., Oncogene 16, 133-1542 (1998)); Tyrphostins AG 1295, AG 1296 (see Kovalenko et al., Cancer Res. 54, 6106-6114 (1994), Lipson et al., Pharmacol & Exp- Therap. 285, 844-852 (1998), Krystal et al., Cancer Res. 57, 2203-2208 (1997)); SU 101 (Leflunomide), as well as its metabolite (see Shawer et al., Clin. Cancer Res. 3,1167-1177 (1997), Mattar et al., FEBS Lett 334, 161-164 (1993), Cherwinskyi et al., Inflamm. Res. 3, 1167-1177 (1997), and Strawn et al., Exp. Opin. Invest. Drugs 7, 533-573 (1998)); and Pyridopyrimidines (see e.g. Hamby et al., J. Med. Chem. 40, 2296-2303 (1997), Dahring et al., J. Pharmacol. Exp, Ther. 281. 1446-1456 (1997), Klutcho et al., Life Sci. 62,143-150 (1998), Panek et al., J. Pharmacol. Exp. Ther. 283; 1433-1444 (1997), Boschelli et al., J. Med. Chem. 41, 4365-4377 (1998)). The tyrosine kinase inhibitors, their synthesis and their use can be deduced from these reference.

The term "and/or" used in "abl-, PDGF-R- and/or Kit receptor-tyrosine kinase inhibitor" means that either one or more of the mentioned tyrosine kinases is inhibited by a compounds encompassed by this expression.

Preferably, one of the following compounds is meant by the term "abl-, PDGF-R- and/or Kit receptor-tyrosine kinase inhibitor":
(A) A compound of the formula IV, 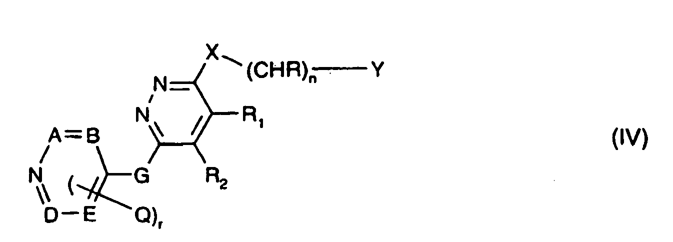 wherein
   r is 0 to 2;
   n is 0 to 2;
   m is 0 to 4;
   R₁ and R₂ (i) are lower alkyl, especially methyl, or
   (ii) together form a bridge in subformula I* 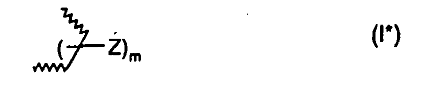 the binding being achieved via the two terminal carbon atoms, or
   (iii) together form a bridge in subformula I** 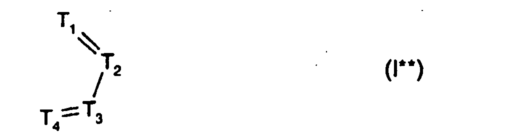 wherein one or two of the ring members T₁, T₂, T₃ and T₄ are nitrogen, and the others are in each case CH, and the binding is achieved via T₁ and T₄;
   A, B, D, and E are, independently of one another, N or CH, with the stipulation that not more than 2 of these radicals are N;
   G is lower alkylene, lower alkylene substituted by acyloxy or hydroxy, -CH₂O-, -CH₂-S-, -CH₂-NH-, oxa (-O-), thia (-S-), or imino (-NH-);
   Q is lower alkyl, especially methyl;
   R is H or lower alkyl;
   X is imino, oxa, or thia;
   Y is aryl, pyridyl, or unsubstituted or substituted cycloalkyl; and
   Z is mono- or disubstituted amino, halogen, alkyl, substituted alkyl, hydroxy, etherified or esterified hydroxy, nitro, cyano, carboxy, esterified carboxy, alkanoyl, carbamoyl, N-mono- or N,N-disubstituted carbamoyl, amidino, guanidino, mercapto, sulfo, phenylthio, phenyl lower alkylthio, alkylphenylthio, phenylsulfinyl, phenyl-lower alkylsulfinyl, alkylphenylsulfinyl, phenylsulfonyl, phenyl-lower alkylsulfonyl, or alkylphenylsulfonyl, wherein - if more than 1 radical Z (m = ≥ 2) is present - the substituents Z are the same or different from one another;
   and wherein the bonds characterized, if present, by a wavy line are either single or double bonds;
   or an N-oxide of the defined compound, wherein 1 or more N atoms carry an oxygen atom;
   with the stipulation that, if Y is pyridyl or unsubstituted cycloalkyl, X is imino, and the remaining radicals are as defined, G is selected from the group comprising lower alkylene, - CH₂-O-, -CH₂-S-, oxa and thia;
   or a salt thereof.
   Preferably, the definitions of the substituents given above have the meanings, especially the preferred meanings, described in International Patent Application WO 98/35958. Most preferred of these compounds is the compound of the formula V 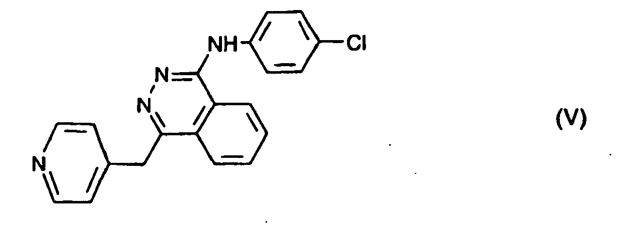 with the name 1-(4-chloro-anilino)-4-(4-pyridyl-methyl)-phthalazine, or a pharmaceutically acceptable salt thereof;
(B) a 7H-pyrrolo[2,3-d]pyrimidine compound of the formula VI 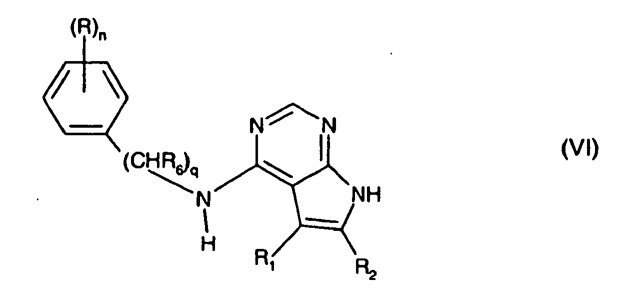 wherein
   q is 0 or 1,
   n is from 1 to 3 when q is 0, or n is from 0 to 3 when q is 1,
   R is halogen, lower alkyl, hydroxy, lower alkanoyloxy, lower alkoxy, carboxy, lower alkoxycarbonyl, carbamoyl, N-lower alkyl-carbamoyl, N,N-di-lower alkyl-carbamoyl, cyano, amino, lower alkanoylamino, lower alkylamino, N,N-di-lower alkylamino or tri-fluoromethyl, it being possible when several radicals R are present in the molecule for those radicals to be identical or different;
   a) R₁ and R₂ are each independently of the other
      α) phenyl substituted by carbamoyl-methoxy, carboxy-methoxy, benzoyloxycarbonyl-methoxy, lower-alkoxycarbonyl-methoxy, phenyl, amino, lower alkanoylamino, lower alkylamino, N,N-di-lower alkylamino, hydroxy, lower alkanoyloxy, carboxy, lower alkoxycarbonyl, carbamoyl, N-lower alkylcarbamoyl, N,N-di-lower alkyl-carbamoyl, cyano or by nitro;
      β) hydrogen;
      γ) unsubstituted or halo- or lower alkyl-substituted pyridyl;
      δ) N-benzyl-pyridinium-2-yl; naphthyl; cyano; carboxy; lower alkoxycarbonyl; carbamoyl; N-lower alkylcarbamoyl; N,N-di-lower alkylcarbamoyl,; N-benzyl-carbamoyl; formyl; lower alkanoyl; lower alkenyl; lower alkenyloxy; or
      ε) lower alkyl substituted by
      εα) halogen, amino, lower alkylamino, piperazino, di-lower alkylamino,
      εβ) phenylamino that is unsubstituted or substitutedin the phenyl moiety by halogen, lower alkyl, hydroxy, lower alkanoyloxy, lower alkoxy, carboxy, lower alkoxycarbonyl, carbamoyl, N-lower alkylcarbamoyl, N,N-di-lower alkylcarbamoyl, cyano, amino, lower alkanoylamino, lower alkylamino, N,N-di-lower alkylamino or by trifluoromethyl,
      εγ) hydroxy, lower alkoxy, cyano, carboxy, lower alkoxycarbonyl, carbamoyl, N-lower alkylcarbamoyl, N,N-di-lower alkyl-carbamoyl, mercapto, or
      εδ) by a radical of the formula R₃-S(O)ₘ- wherein R₃ is lower alkyl and m is 0, 1 or 2, or
   b) when q is 1, one of the radicals R₁ and R₂ is unsubstituted lower alkyl or unsubstituted phenyl and the other of the radicals R₁ and R₂ has one of the meanings given above in paragraph a) with the exception of hydrogen, or
   c) R₁ and R₂ together are C₄-C₁₀-1,4-alkadienylene substituted by amino, lower alkanoylamino, lower alkylamino, N,N-di-lower alkylamino, nitro, halogen, hydroxy, lower alkanoyloxy, carboxy, lower alkoxycarbonyl, carbamoyl, N-lower alkylcarbamoyl, N,N-di-lower alkyl-carbamoyl or by cyano, or are aza-1,4-alkadienylene having up to 9 carbon atoms, or
   d) when q is 1, R₁ and R₂ are, each independently of the other, unsubstituted lower alkyl or unsubstituted phenyl or have one of the meanings given above in paragraph a), and
   R₆ is hydrogen, lower alkyl, lower alkoxycarbonyl, carbamoyl, N-lower alkyl-carbamoyl or N,N-di-lower alkyl-carbamoyl,
   or a salt thereof.
   Preferably, the definitions of the substituents given above have the meanings, especially the preferred meanings, described in International Patent Application WO 97/02266. Most preferred of these compounds is the compound of the formula VII 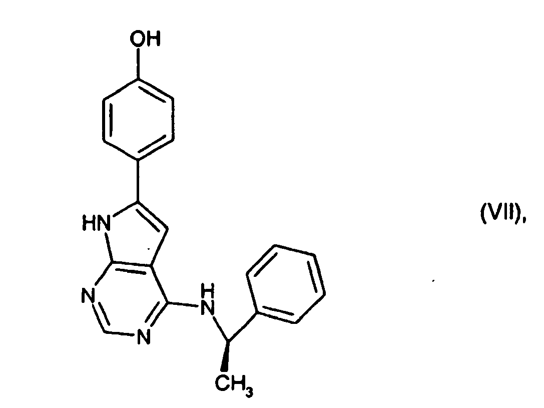 having the name (R)-6-(4-hydroxy-phenyl)-4-[(1-phenylethyl)-amino]-7H-pyrrolo[2,3-d]pyrimidine; or most preferred
(C) an N-phenyl-2-pyrimidine-amine derivative of formula I 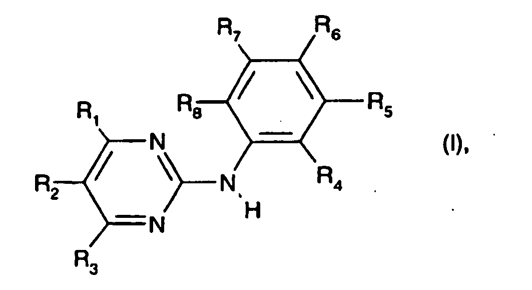 wherein
   R₁ is pyrazinyl, 1-methyl-1H-pyrrolyl, amino- or amino-lower alkyl-substituted phenyl wherein the amino group in each case is free, alkylated or acylated, 1 H-indolyl or 1 H-imidazolyl bonded at a five-membered ring carbon atom, or unsubstituted or lower alkyl-substituted pyridyl bonded at a ring carbon atom and unsubstituted or substituted at the nitrogen atom by oxygen,
   R₂ and R₃ are each independently of the other hydrogen or lower alkyl,
   one or two of the radicals R₄, R₅, R₆, R₇ and R₈ are each nitro, fluoro-substituted lower alkoxy or a radical of formula II

   -N(R₉)-C(=X)-(Y)ₙ-R₁₀ (II),

   wherein
   - R₉: is hydrogen or lower alkyl,
   - X: is oxo, thio, imino, N-lower alkyl-imino, hydroximino or O-lower alkyl-hydroximino,
   - Y: is oxygen or the group NH,
   - n: is 0 or 1 and
   - R₁₀: is an aliphatic radical having at least 5 carbon atoms, or an aromatic, aromatic-aliphatic, cycloaliphatic, cycloaliphatic-aliphatic, heterocyclic or heterocyclic-aliphatic radical, and the remaining radicals R₄, R₅, R₆, R₇ and R₈ are each independently of the others hydrogen, lower alkyl that is unsubstituted or substituted by free or alkylated amino, piperazinyl, piperidinyl, pyrrolidinyl or by morpholinyl, or lower alkanoyl, trifluoromethyl, free, etherified or esterifed hydroxy, free, alkylated or acylated amino or free or esterified carboxy, or
   a salt of such compounds having at least one salt-forming group.

Preferably, the definitions of the substituents given above have the meanings, especially the preferred meanings, described in European Patent Application EP 0 564 409. Most preferred of these compounds is the compound of the formula III 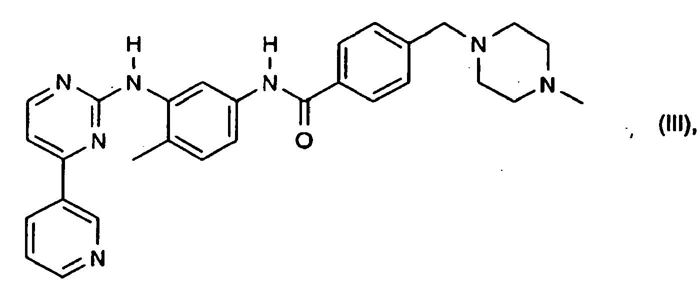 with the name 4-(4-methylpiperazin-1-ylmethyl)-N-(4-methyl-3-(4-pyridin-3-yl)pyrimidin-2-ylamino)phenyl]benzamide, preferably in the form of the methane sulfonate salt as described in WO 99/03854, most preferably in the form of the methane sulfonate salt in the β-crystal form as described in WO 99/03854. This compound (the methane sulfonate salt form) is called STI571 hereinafter.

An "organic compound capable of binding to α₁-acidic glycoprotein (AGP)" is generally a basic or neutral drug, but also an acidic drug, especially selected from the group consisting of
alpha-blockers, especially Nicergoline or Prazosin;
anesthetics/analgesics, especially Alfentanil, Ketamine or Ethidocaine;
analgetics, especially Fentanil, Meperidine, Methadone or Phenylbutazone;
anesthetics, especially Bupivacaine, Etidocaine or Phencyclidine;
anesthetics/antiarrhytmics, especially Lidocaine or Phencyclidin;
antiarrhytmics, especially Aprindine, Disopyramide, Quinidine or Verapamil;
antibiotics, especially Erythromycin;
anticoagulants, especial Acenocoumarol, Dipyridamole, PCR2362 (thienopyridine derivative), Ticlopidine or Warfarin;
antiepileptics, especially Phenytoin or Carbamazepine;
antiinflammatory agents, especially Naproxen;
beta-blockers, especially Alprenolol, Metoprolol, Oxprenolol, Pindolol and related compounds, Propranolol or Timolol;
steroids, such as Progesterone, Cortexone, Cortisol, Testosteron, Estradiol.or Prednisolone; neuromuscular blockers, especially Metocurine or d-Tubocurarine;
psychotropics, especially Amitriptyline, Chlorpromazine, Cyclazindol, Desmethylimipramine, Diazepam, Doxepine, Flurazepam, Fluphenazine, Haloperidol, Imipramine, Loxapine, Mianserin, Nortriptyline, Norzimelidine, Perazine, Perphenazine, Phenobarbital, Phenothiazine derivatives, Promazine, Acepromazine, Protipendyl, Thioridazine, Thiothixene, Triazolam, Trifluoperazine or Zimelidine;
vitamins and provitamins, especially Vitamin B₁₂ or folic acid;
fluorescent probes, especially DAPN (derivative of propranolol), 1,8-Anilino-naphthalene sulfonate;
further drugs, especially Aminopyrine, Amoxapine, Bupropion, Maprotiline, Nomifensine, Trazodone, drugs with quaternary ammonium group, Ritodrine, Doxazosin, Trimazosin, Binedalin, Amsacrine, Apazone, SKF 525A, Ciclazindol, PCR 2362, Indomethacin, Probenecid, Retinoic Acid, Sulfinpyrazone, Tolmetin, Benoxaprofen, Heparin, Sufentanil, Lofentanil, Metoclopramide, Nicardipine, Pirmenol, mifepristone, RU 42 633, Aprindil, Auramine O, Bepridil, Desipramine, Desmethylclomipramine, Moxaprindine, Quinine, Lorcainide, Prothipendyl, Protriptyline, Trihexyphenidyl, Biperiden, Methaqualone, Diphenhydramine, Glutethimide, Chlordiazepoxid, L-Tryptophane, Mepivacaine, Levomethadone, Opipramol, Trifluopromazine or Trimipramine;
plasticicers, such as tris-butoxyethyl phosphate (TBEP);
staurosporine (see US 4,107,297) or staurosporine derivatives, preferably those disclosed in European Patent Application EP 0 296 110 and/or EP 0 238 011, especially N-benzoyl-staurosporine (PTK412 - see European Patent Application No. EP 0 296 110) or 7-hydroxy staurosporine (UCN-01 - see European Patent Application No. EP 0 238 011;
as well as a metabolite of any of these compounds;
or a - especially pharmaceutically acceptable - salt thereof.

EP 0 238 011, US 4,107,297 and EP 0 296 110; Kremer et al., Pharmacol. Rev. 40(1), 1 - 47 (1988); Cancer Res. 58, 3248-3253 (1998); Br. J. Clin. Pharmacol. 22, 499-506 (1986); Physiol. Functions, and Pharmacol., pages 321-336: F. Bree et al., "Binding to α1-acidic glycoprotein and relevant apparent volume of distribution", Alan R. Liss Inc., 1989; Protein Binding and Drug Transport (Tillement and Lindenlaub, eds.): "Drug binding to human α1-acidic glycoprotein - focus on a single binding site", Stuttgart 1986; In all of these publications, compounds capable of binding to AGP are mentioned which are preferred embodiments of the present invention.

More preferred is any of the compounds mentioned above other than a steroid. For second medical use (e.g. in patient groups where resistance has developed, more especially CML patients in blast crisis and relapsed Ph+-ALL patients), also steroids are useful.

An organic compound capable of binding to AGP to be used in the combination according to the invention in addition to an abl-, PDGF-R- or Kit receptor-tyrosine kinase may also be selected from one or more additional abl-, PDGF-R- or Kit receptor-tyrosine kinase(s), meaning that component (b) in the embodiments of the invention may also be such a compound.

Preferably, the abl-, PDGF-R- and/or kit receptor-tyrosine kinase (component (a)) is selected from the group consisting of 1-(4-chloro-anilino)-4-(4-pyridyl-methyl)-phthalazine, (R)-6-(4-hydroxy-phenyl)-4-[(1-phenylethyl)-amino]-7H-pyrrolo[2,3-d]pyrimidine and preferably 4-(4-methylpiperazin-1-ylmethyl)-N-[4-methyl-3-(4-pyridin-3-yl)pyrimidin-2-ylamino)phenyl]-benzamide; or a salt thereof; more preferably the methane sulfonate salt of 4-(4-methylpiperazin-1-ylmethyl)-N-[4-methyl-3-(4-pyridin-3-yl)pyrimidin-2-ylamino)phenyl]-benzamide, most preferably in the β-crystal form as described in WO 99/03854.

The organic compound capable of binding to α₁-acidic glycoprotein (component (b)) is preferably an antibiotic, especially Erythromycin, or staurosporine or a staurosporine derivative, especially N-benzoyl-staurosporine (PKC412) or 7-hydroxy-staurosporine (UCN-01), or a salt thereof, most especially Erythromycin, or a pharmaceutically acceptable salt thereof.

One or more each of the tyrosine kinase inhibitor and the organic compound capable of binding to AGP can be used in a combination or combination therapy according to the present invention.

Binding means especially competitive binding, but may also be any other type of binding (e.g. to binding sites that show allosteric effects on the site binding component (a)) that leads to diminished binding of an abl-, PDGF-R- and/or Kit receptor-tyrosine kinase inhibitor to AGP. This binding may be determined *in vitro* in analogy to the methods described in the Examples hereinbelow.

Binding may be irreversible (e.g. by covalent or ionic binding) or preferably reversible.

"Capable of binding" means that the compound has an affinity to AGP that allows for binding as described above, preferably with a concentration at half-maximal binding in the millimolar to sub-nanomolar range, especially in the range from 100 µM to 1 nM.

By the term "a proliferative disease that can be treated by administration of an abl-, PDGF-R- and/or Kit receptor-tyrosine kinase" any disease mentioned herein is meant; preferably any disease is meant that responds to such compounds; especially, a proliferative disease selected from a cancer disease, especially a tumor disease or leukemia, or a non-malignant proliferative disease, e.g. atherosclerosis, thrombosis, psoriasis, sclerodermitis or fibrosis, is meant. More preferably, a disease selected from the group consisting of CML (chronic myeloid leukemia) and ALL (acute lymphoblastic leukemia), or a solid tumor, especially selected from lung cancer, especially non-small cell lung cancer, and from cancer of the prostate, is meant.

By the term "quantity which is jointly therapeutically effective against a proliferative disease that can be treated by administration of an abl-, PDGF-R- and/or Kit receptor-tyrosine kinase inhibitor" there is preferably meant any quantity of the components of the combinations that, in the combination, is diminishing proliferation of cells responsible for any of the mentioned proliferative diseases (e.g. diminished tumor growth) or, preferably, even causing regression, more preferably even the partial or complete disappearance, of such cells (e.g. tumor regression, preferably cure).

Preferably, in any of the embodiments of the present invention the dose of each of the components of the combination (component (a) and (b)) is chosen so that a blood plasma level that is above the concentration of half-maximal binding of component (b), the organic compound capable of binding to AGP, is achieved *in vivo* at least a part of the time when component (a), the abl-, PDGF-R- and/or Kit receptor-tyrosine kinase inhibitor, is present. That concentration can be determined e.g. *in vitro* according to standard procedures, e.g. in analogy to the methods described in the examples for the determination of Ka, while the blood plasma concentration of components (a) and (b) in a warm-blooded animal (meaning especially a human patient) may also be determined according to routine methods.

Preferably, the dosing of component (b) is chosen so that the concentration of the component (b) is more than 0.05 µM in plasma, more preferably more than 0.1 µM, and most preferably between 0.5 and 100 µM, especially between 1 and 50 µM, of the treated individual, at least part of the time where component (a) is also present. Most preferably, the concentration of any of component (a) and (b) is more than 0.1 µM, more preferably more than 1 µM, and most preferably between 0.5 and 100 µM, especially between 1 and 50 µM, in the blood plasma of the treated individual, at least part of the time where component (a) or component (b), respectively, is also present.

The plasma concentrations can be determined according to standard methods, e.g. employing HPLC of samples worked up according to standard procedures.

By the term "a product which comprises
(a) at least one abl-, PDGF-R- and/or Kit receptor-tyrosine kinase inhibitor and
(b) at least one organic compound capable of binding to α₁-acidic glycoprotein (AGP),
wherein any component (a) and/or (b) can also be present in the form of a pharmaceutically acceptable salt, if at least one salt-forming group is present,
in the presence or absence of one or more pharmaceutically acceptable carrier materials, as a combination preparation for simultaneous or chronologically staggered use within a period of time which is small enough for the active compounds both of component (a) and of component (b) to enhance antiproliferative activity of compound (a) against proliferating cells, especially in a patient, for treating a proliferative disease which responds to such a compound", there is meant especially a "kit" or "kit of parts" in the sense that the effective components (a) and (b) of the combination can be dosed independently or by use of different fixed combinations with distinguished amounts of any components (a) and (b) at different time points. The parts of the kit of parts can then be administered simultaneously or in a chronologically staggered manner, that is at different time points and with equal or different time intervals for any part of the kit of parts, with the condition that the time intervals are chosen such that the effect on the proliferative disease in the combined use of the parts is larger than the effect which would be obtained by use of only component (a), that is, stronger inhibition of proliferation or, preferably, stronger regression or even cure of the proliferative disease is found than when the same dose of only component (a) is administered alone. That is meant by the term "to enhance antiproliferative activity against proliferating cells, especially in a patient"; preferably, there is meant an enhancing of the effect by component (b), especially a partial or complete reversal of resistance of a proliferative disease to one or more compounds of the component (a) type and/or the causing of regression of the proliferating cells, up to and including their complete destruction.

By the term "proliferating cells", preferably abnormally proliferating cells are meant, such as cancer cells.

Preferred are combinations which show enhanced antiproliferative activity when compared with the single component (a) alone, especially combinations that show synergism (synergistic combinations) or combinations that lead to regression of proliferative tissues and/or cure from proliferative diseases, most preferably combinations where a resistance (meaning resistance already at the start of the treatment, or resistance that is a result of more or less extended periods of treatment with component (a)) of a proliferative disease in a warm-blooded animal, especially a human, to one or more compounds of the component (a) type is partially or completely overcome.

The "pharmaceutically acceptable carrier materials" are explained below in the definition of pharmaceutical preparations.

In any combination or combination treatment according to the invention described herein, the use in combination in order to completely or partially reverse resistance (present before treatment or developed or developing during treatment with a drug comprising a component (a) as defined herein) of a proliferative disease to treatment with a drug comprising a component (a) as defined herein *in vivo,* especially in a warm-blooded animal, especially a human, is preferred.

Complete or partial resistance especially means that a lower efficiency, e.g. in terms of stopping or delaying of proliferation, causing of regression or even cure, e.g. less proliferation inhibition or a longer duration of treatment until a response expected if no resistance were present, e.g. of a tumor or leukemia, is found than in an animal, e.g. human, not showing resistance, or that initial treatment successes (especially in a patient that develops resistance only during treatment with a component (a)) are no longer found at later stages of treatment, especially in CML patients in blast crisis and relapsed Ph+-ALL patients.

It is to be understood that the invention relates also to any use of combinations of a component (a) and a component (b), as defined above and below, in a method of treatment of cells outside the body with the intent to replace hyperproliferating cells in the body of a subject with hyperproliferating cells by normal cells; for example, blood with cells of the immune system may be taken from a subject, treated outside the body with a component (a) and a component (b) to select for non-hyperproliferative cells, the stem cells and the remaining blood cells of the immune system may be destroyed in the subject e.g. by irradiation or chemotherapy and then the selected normal cells may be reimplanted into the subject, e.g. by injection etc. The methods to be employed in such kinds of treatment are known to the person having skill in the art.

Provided that one or more salt-forming groups are present, the drug substances corresponding to component (a) and/or (b) may also be present in the form of salts.

Salts are especially the pharmaceutically acceptable, e.g. substantially non-toxic, salts.

Such salts are formed, for example, from compounds having an acidic group, for example a carboxy, phosphodiester or phosphorothioate group, and are, for example, their salts with suitable bases, such as non-toxic metal salts derived from metals of groups Ia, Ib, IIa and IIb of the Periodic Table of Elements, especially suitable alkali metal salts, for example lithium, sodium or potassium salts, or alkaline earth metal salts, for example magnesium or calcium salts, furthermore zinc or ammonium salts, also those salts that are formed with organic amines, such as unsubstituted or hydroxy-substituted mono-, di- or tri-alkylamines, especially mono-, di- or tri-lower alkylamines, or with quaternary ammonium compounds, for example with N-methyl-N-ethylamine, diethylamine, triethylamine, mono-, bis- or tris-(2-hydroxy-lower alkyl)amines, such as mono-, bis- or tris-(2-hydroxyethyl)amine, 2-hydroxy-tert-butylamine or tris(hydroxymethyl)methylamine, N,N-di-lower alkyl-N-(hydroxy-lower alkyl)-amines, such as N,N-dimethyl-N-(2-hydroxyethyl)-amine or tri-(2-hydroxyethyl)-amine, or N-methyl-D-glucamine, or quaternary ammonium salts, such as tetrabutylammonium salts. Compounds having a basic group, for example an amino or imino group, can form acid addition salts, for example with inorganic acids, for example a hydrohalic acid, such as hydrochloric acid, sulfuric acid or phosphoric acid, or with organic carboxylic, sulfonic, sulfo or phospho acids or N-substituted sulfamic acids, such as, for example, acetic acid, propionic acid, glycolic acid, succinic acid, maleic acid, hydroxymaleic acid, methyl maleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid or isonicotinic acid, also with amino acids, for example, α-amino acids, and also with methanesulfonic acid, ethanesulfonic acid, 2-hydroxyethanesulfonic acid, ethane-1,2-disulfonic acid, benzenesulfonic acid, 4-methylbenzenesulfonic acid, naphthalene-2-sulfonic acid, 2- or 3-phosphoglycerate, glucose-6-phosphate, N-cyclohexylsulfamic acid (with formation of the cyclamates) or with other acidic organic compounds, such as ascorbic acid. Compounds having acidic and basic groups can also form internal salts. If more than one salt-forming group is present, it is also possible that mixed salts are present.

For the purpose of isolation or purification, it is also possible to use pharmaceutically unacceptable salts, for example picrate or perchlorate salts.

The terms "compounds" "components" and "salts" also expressly include individual compounds or individual salts.

As can be understood from the present text, the term "combination" in the preceding paragraphs and especially in the following paragraphs which describe more specific and preferred variants of the present invention is intended to refer to
(i) a combination preparation comprising (a) at least one abl-, PDGF-R- and/or Kit receptor-tyrosine kinase inhibitor and (b) at least one organic compound capable of binding to α₁-acidic glycoprotein (AGP); or pharmaceutically acceptable salts of any component (a), (b) or (a) and (b) if at least one salt-forming group is present;
(ii) a method for treating a proliferative disease that can be treated by administration of an abl-, PDGF-R- and/or Kit receptor-tyrosine kinase inhibitor, wherein (a) at least one abl-, PDGF-R- and/or Kit receptor-tyrosine kinase inhibitor and
   (b) at least one organic compound capable of binding to α₁-acidic glycoprotein (AGP) are administered to a mammal in combination in a quantity which is jointly therapeutically effective against a proliferative disease that can be treated by administration of an abl-, PDGF-R- and/or Kit receptor-tyrosine kinase inhibitor, wherein any component (a) and/or (b) can also be present in the form of a pharmaceutically acceptable salt, if at least one salt-forming group is present;
(iii) a product which comprises
   (a) at least one abl-, PDGF-R- and/or Kit receptor-tyrosine kinase inhibitor and
   (b) at least one organic compound capable of binding to α₁-acidic glycoprotein (AGP),
   wherein any component (a) and/or (b) can also be present in the form of a pharmaceutically acceptable salt, if at least one salt-forming group is present,
   in the presence or absence of one or more pharmaceutically acceptable carrier materials, as a combination preparation for simultaneous or chronologically staggered use within a period of time which is small enough for the active compounds both of component (a) and of component (b) to enhance antiproliferative activity of compound (a) against proliferating cells, especially in a patient, for treating a proliferative disease which responds to such a compound;
(iv) a pharmaceutical preparation which comprises a quantity, which is jointly effective for treating a proliferative disease that can be treated by administration of an abl-, PDGF-Rand/or Kit receptor-tyrosine kinase inhibitor, of
   (a) at least one abl-, PDGF-R- and/or Kit receptor-tyrosine kinase inhibitor and
   (b) at least one organic compound capable of binding to α₁-acidic glycoprotein,
      wherein any component (a) and/or (b) can also be present in the form of a pharmaceutically acceptable salt, if at least one salt-forming group is present, with one or more pharmaceutically acceptable carrier materials; and/or
(v) the use of a combination of
   (a) at least one abl-, PDGF-R- and/or Kit receptor-tyrosine kinase inhibitor and
   (b) at least one organic compound capable of binding to α₁-acidic glycoprotein,
   wherein any component (a) and/or (b) can also be present in the form of a pharmaceutically acceptable salt, if at least one salt-forming group is present,
   for producing pharmaceutical preparations for use as compositions against a proliferative disease that can be treated by administration of an abl-, PDGF-R- and/or Kit receptor-tyrosine kinase inhibitor;
   or any combination of these subjects of the invention, as far as permissible under the respective patent laws;
   or the more specific and preferred variants thereof as given below;
   wherein any component (a) and/or (b) can also be present in the form of a pharmaceutically acceptable salt, if at least one salt-forming group is present;
   if not defined otherwise or evident otherwise from the context.

Within the following groups of more preferred embodiments of the invention, more general definitions may be replaced by more specific definitions in accordance with those given above or (especially with regard to definition of pharmaceutical compositions and methods of use) below.

Preferred is a combination of (a) at least one, preferably one, abl-, PDGF-Receptor and/or Kit receptor-tyrosine kinase inhibitor selected from
(i) a 7H-pyrrolo[2,3-d]pyrimidine compound of the formula VI 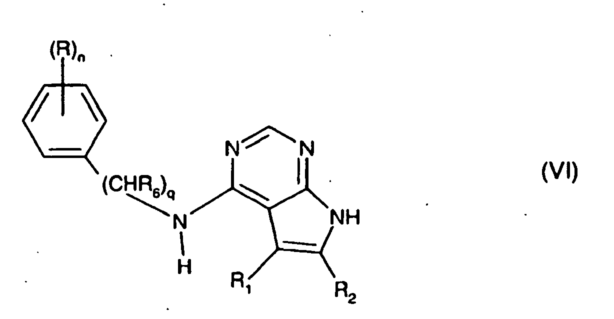 wherein
   q is 0 or 1,
   n is from 1 to 3 when q is 0, or n is from 0 to 3 when q is 1,
   R is halogen, lower alkyl, hydroxy, lower alkanoyloxy, lower alkoxy, carboxy, lower alkoxycarbonyl, carbamoyl, N-lower alkyl-carbamoyl, N,N-di-lower alkyl-carbamoyl, cyano, amino, lower alkanoylamino, lower alkylamino, N,N-di-lower alkylamino or tri-fluoromethyl, it being possible when several radicals R are present in the molecule for those radicals to be identical or different;
   a) R₁ and R₂ are each independently of the other
      α) phenyl substituted by carbamoyl-methoxy, carboxy-methoxy, benzoyloxycarbonyl-methoxy, lower-alkoxycarbonyl-methoxy, phenyl, amino, lower alkanoylamino, lower alkylamino, N,N-di-lower alkylamino, hydroxy, lower alkanoyloxy, carboxy, lower alkoxycarbonyl, carbamoyl, N-lower alkylcarbamoyl, N,N-di-lower alkyl-carbamoyl, cyano or by nitro;
      hydrogen;
      γ) unsubstituted or halo- or lower alkyl-substituted pyridyl;
      δ) N-benzyl-pyridinium-2-yl; naphthyl; cyano; carboxy; lower alkoxycarbonyl; carbamoyl; N-lower alkylcarbamoyl; N,N-di-lower alkylcarbamoyl; N-benzyl-carbamoyl; formyl; lower alkanoyl; lower alkenyl; lower alkenyloxy; or
      ε) lower alkyl substituted by
      εα) halogen, amino, lower alkylamino, piperazino, di-lower alkylamino,
      εβ) phenylamino that is unsubstituted or substitutedin the phenyl moiety by halogen, lower alkyl, hydroxy, lower alkanoyloxy, lower alkoxy, carboxy, lower alkoxycarbonyl, carbamoyl, N-lower alkylcarbamoyl, N,N-di-lower alkylcarbamoyl, cyano, amino, lower alkanoylamino, lower alkylamino, N,N-di-lower alkylamino or by trifluoromethyl,
      εγ) hydroxy, lower alkoxy, cyano, carboxy, lower alkoxycarbonyl, carbamoyl, N-lower alkylcarbamoyl, N,N-di-lower alkyl-carbamoyl, mercapto, or
      εδ) by a radical of the formula R₃-S(O)ₘ- wherein R₃ is lower alkyl and m is 0, 1 or 2, or
   b) when q is 1, one of the radicals R₁ and R₂ is unsubstituted lower alkyl or unsubstituted phenyl and the other of the radicals R₁ and R₂ has one of the meanings given above in paragraph a) with the exception of hydrogen, or
   c) R₁ and R₂ together are C₄-C₁₀-1,4-alkadienylene substituted by amino, lower alkanoylamino, lower alkylamino, N,N-di-lower alkylamino, nitro, halogen, hydroxy, lower alkanoyloxy, carboxy, lower alkoxycarbonyl, carbamoyl, N-lower alkylcarbamoyl, N,N-di-lower alkyl-carbamoyl or by cyano, or are aza-1,4-alkadienylene having up to 9 carbon atoms, or
   d) when q is 1, R₁ and R₂ are, each independently of the other, unsubstituted lower alkyl or unsubstituted phenyl or have one of the meanings given above in paragraph a), and

R₆ is hydrogen, lower alkyl, lower alkoxycarbonyl, carbamoyl, N-lower alkyl-carbamoyl or N,N-di-lower alkyl-carbamoyl,
or a salt thereof (preferably, the definitions of the substituents given above have the meanings, especially the preferred meanings, described in International Patent Application WO 97/02266 - most preferred of these compounds is the compound of the formula VII 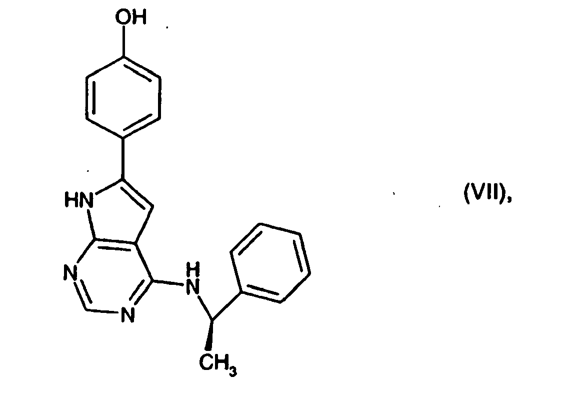 having the name (R)-6-(4-hydroxy-phenyl)-4-[(1-phenylethyl)-amino]-7H-pyrrolo[2,3-d]pyrimidine);
and
(ii) an N-phenyl-2-pyrimidine-amine derivative of formula I 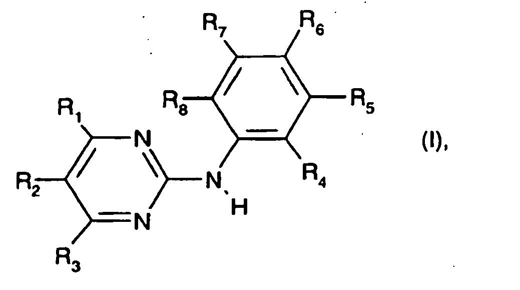 wherein
R₁ is pyrazinyl, 1-methyl-1H-pyrrolyl, amino- or amino-lower alkyl-substituted phenyl wherein the amino group in each case is free, alkylated or acylated, 1 H-indolyl or 1 H-imidazolyl bonded at a five-membered ring carbon atom, or unsubstituted or lower alkyl-substituted pyridyl bonded at a ring carbon atom and unsubstituted or substituted at the nitrogen atom by oxygen,
R₂ and R₃ are each independently of the other hydrogen or lower alkyl,
one or two of the radicals R₄, R₅, R₆, R₇ and R₈ are each nitro, fluoro-substituted lower alkoxy or a radical of formula II

-N(R₉)-C(=X)-(Y)ₙ-R₁₀ (II),

wherein
- R₉: is hydrogen or lower alkyl,
- X: is oxo, thio, imino, N-lower alkyl-imino, hydroximino or O-lower alkyl-hydroximino,
- Y: is oxygen or the group NH,
- n: is 0 or 1 and
- R₁₀: is an aliphatic radical having at least 5 carbon atoms, or an aromatic, aromatic-aliphatic, cycloaliphatic, cycloaliphatic-aliphatic, heterocyclic or heterocyclic-aliphatic radical, and the remaining radicals R₄, R₅, R₆, R₇ and R₈ are each independently of the others hydrogen, lower alkyl that is unsubstituted or substituted by free or alkylated amino, piperazinyl, piperidinyl, pyrrolidinyl or by morpholinyl, or lower alkanoyl, trifluoromethyl, free, etherified or esterifed hydroxy, free, alkylated or acylated amino or free or esterified carboxy, or
a salt of such compounds having at least one salt-forming group (preferably, the definitions of the substituents given above have the meanings, especially the preferred meanings, described in European Patent Application EP 0 564 409. Most preferred of these compounds is the compound of the formula III 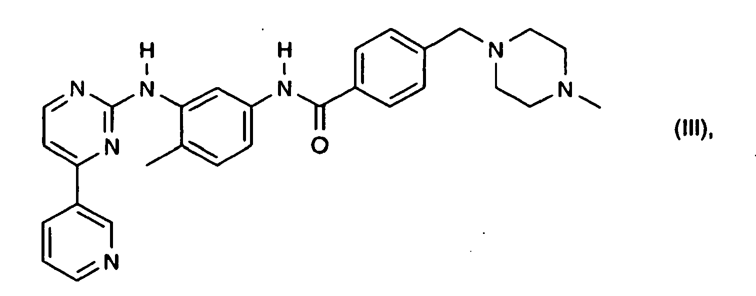 with the name 4-(4-methylpiperazin-1-ylmethyl)-N-[4-methyl-3-(4-pyridin-3-yl)pyrimidin-2-ylamino)phenyl]benzamide, preferably in the form of the methane sulfonate salt as described in WO 99/03854, most preferably in the form of the methane sulfonate salt in the β-crystal form as described in WO 99/03854);
with (b) at least one (preferably one) organic compound capable of binding to α₁-acidic glycoprotein (AGP) selected from the group consisting of:
Nicergoline, Prazosin, Alfentanil, Ketamine, Ethidocaine, Fentanil, Meperidine, Methadone, Phenylbutazone, Bupivacaine, Etidocaine, Phencyclidine, Lidocaine, Phencyclidin, Aprindine, Disopyramide, Quinidine, Verapamil, Erythromycin, Acenocoumarol, Dipyridamole, PCR2362, Ticlopidine, Warfarin, Phenytoin, Carbamazepine, Naproxen, Alprenolol, Metoprolol, Oxprenolol, Pindolol, Propranolol, Timolol, Progesterone, Cortexone, Cortisol, Testosteron, Estradiol, Prednisolone, Metocurine, d-Tubocurarine, Amitriptyline, Chlorpromazine, Cyclazindol, Desmethylimipramine, Diazepam, Doxepine, Flurazepam, Fluphenazine, Haloperidol, Imipramine, Loxapine, Mianserin, Nortriptyline, Norzimelidine, Perazine, Perphenazine, Phenobarbital, Phenothiazine derivatives, Promazine, Acepromazine, Protipendyl, Thioridazine, Thiothixene, Triazolam, Trifluoperazine, Zimelidine, Vitamin B₁₂, folic acid,
DAPN, 18-Anilino-naphthalene sulfonate, Aminopyrine, Amoxapine, Bupropion, Maprofiline, Nomifensine, Trazodone, Ritodrine, Doxazosin, Trimazosin, Binedalin, Amsacrine, Apazone, SKF 525A, Ciclazindol, PCR 2362, Indomethacin, Probenecid, Retinoic Acid, Sulfinpyrazone, Tolmetin, Benoxaprofen, Heparin, Sufentanil, Lofentanil, Metoclopramide, Nicardipine, Pirmenol, mifepristone, RU 42 633, Aprindil, Auramine O, Bepridil, Desipramine, Desmethylclomipramine, Moxaprindine, Quinine, Lorcainide, Prothipendyl, Protriptyline, Trihexyphenidyl, Biperiden, Methaqualone, Diphenhydramine, Glutethimide, Chlordiazepoxid, L-Tryptophane, Mepivacaine, Levomethadone, Opipramol, Trifluopromazine, Trimipramine, tris-butoxyethyl phosphate, staurosporine, N-benzoyl-staurosporine and 7-hydroxy staurosporine;
as well as a metabolite of any of these compounds;
wherein any component (a) and/or (b) can preferably be present in the free form or in the form of a pharmaceutically acceptable salt, if at least one salt-forming group is present.

More preferred is a combination of (a) at least one, preferably one, abl-, PDGF-Receptor-and/or Kit receptor-tyrosine kinase inhibitor selected from the group consisting of 1-(4-chloro-anilino)-4-(4-pyridyl-methyl)-phthalazine, (R)-6-(4-hydroxy-phenyl)-4-[(1-phenylethyl)-amino]-7H-pyrrolo[2,3-d]pyrimidine and preferably 4-(4-methylpiperazin-1-ylmethyl)-N-[4-methyl-3-(4-pyridin-3-yl)pyrimidin-2-ylamino)phenyl]benzamide; or a pharmaceutically acceptable salt of any one or more of these compounds; more preferably the methane sulfonate salt of 4-(4-methylpiperazin-1-ylmethyl)-N-(4-methyl-3-(4-pyridin-3-yl)pyrimidin-2-ylamino)phenyl]benzamide, most preferably in the β-crystal form; with (b) at least one, preferably one, compound capable of binding to α₁-acidic glycoprotein which is preferably an antibiotic, especially Erythromycin, or staurosporine or a staurosporine derivative, especially N-benzoyl-staurosporine or 7-hydroxy-staurosporine, or a pharmaceutically acceptable salt thereof, most especially Erythromycin, or a pharmaceutically acceptable salt thereof.

Most preferably, in all embodiments mentioned above the disease to be treated is a cancer disease, especially a leukemia or a solid tumor, preferably a disease selected from the group consisting of CML (chronic myeloid leukemia) and ALL (acute lymphoblastic leukemia), or a solid tumor, especially selected from lung cancer, especially non-small cell lung cancer, and from cancer of the prostate.

Preferably, a combination according to the present invention is used in the treatment of a warm-blooded animal, especially a human, that has a proliferative disease which (especially due to higher than normal AGP levels) is or, during treatment with an abl-, PDGF-R- and/or Kit receptor-tyrosine kinase inhibitor, is becoming or has become completely or partially resistant to such treatment, such warm-blooded animals representing a special group of probationers.

In another preferred embodiment of the present invention, the combination is used aiming at a warm-blooded animal, especially a human, in order to already prophylactically avoid the emerging of a partial or complete resistance during treatment of a proliferative disease with an abl-, PDGF-R- and/or Kit receptor-tyrosine kinase inhibitor.

### Pharmaceutical Compositions (= Preparations) and Processes:

Where in the following "component (a) and/or (b)" is mentioned, this is intended to mean any one or more of the compounds defined above as component (a) and component (b) as such or a pharmaceutically acceptable salt of one or more of the respective components.

The pharmaceutical compositions that can find use in a combination according to the invention are comprising either one or more of the components (a) and (b) with the properties according to the invention as active ingredient. The combinations can be used alone (e.g. as fixed combination) or as kit of parts. Especially preferred are compositions for enteral, especially oral, or parenteral administration. The compositions comprise one or more of the components (a) and (b) or combinations thereof as such or, preferably, together with a pharmaceutically acceptable carrier. The dose of any active ingredient depends on the disease to be treated and on the species, age, weight and individual condition, as well as the method of administration.

Preferred is a pharmaceutical composition or combination that is suitable for administration to a warm-blooded animal, especially a human, suffering from any disease mentioned herein; preferably any disease is meant that responds to an abl-, PDGF-R- and/or Kit receptor-tyrosine kinase inhibitor; especially, a proliferative disease selected from a cancer disease, especially a tumor disease or leukemia, or a non-malignant proliferative disease, e.g. atherosclerosis, thrombosis, psoriasis, sclerodermitis or fibrosis, is meant; more preferably, a disease selected from the group consisting of CML (chronic myeloid leukemia) and ALL (acute lymphoblastic leukemia), or a solid tumor, especially selected from lung cancer, especially non-small cell lung cancer, and from cancer of the prostate, is meant; most preferably, any of the diseases just mentioned that has become or is becoming resistant to treatment with one or more of the mentioned tyrosine kinase inhibitors, or was resistant to such treatment already before the treatment with any such tyrosine kinase inhibitor, especially due to higher AGP concentrations being present in the blood plasma of the individual to be treated, is meant.

The pharmaceutical compositions comprise from approximately 0.0001 % to approximately 95 % of any component (a) and/or (b), dosage forms that are in single dose form preferably comprising from approximately 10 % to approximately 90 % of component (a) or (b), and dosage forms that are not in single dose form preferably comprising from approximately 10 % to approximately 60 % of each component. Unit dose forms, such as dragées, tablets, ampoules or capsules, comprise from approximately 5 mg to approximately 1.5 g of component (a) and/or component (b), preferably from 5 mg to approximately 1 g.

The pharmaceutical compositions are prepared in a manner known *per* se, for example by means of conventional mixing, granulating, confectioning, dissolving or lyophilising processes. For example pharmaceutical compositions for oral administration can be obtained by combining component (a) and/or (b) with one or more solid or liquid carriers, where necessary granulating a resulting mixture and processing the mixture or the granules, if desired or appropriate with the addition of further excipients, to form tablets or dragée cores or solutions, respectively.

Suitable carriers are especially fillers, such as sugars, e.g. lactose, saccharose, mannitol or sorbitol, cellulose preparations and/or calcium phosphates, e.g. tricalcium phosphate or calcium hydrogen phosphate, and binders, such as starches, e.g. corn, wheat, rice or potato starch, methylcellulose, hydroxypropylmethylcellulose, sodium carboxymethylcellulose and/or polyvinylpyrrolidone, and/or, if desired, disintegrators, such as the above-mentioned starches, and also carboxymethyl starch, crosslinked polyvinylpyrrolidone or alginic acid or a salt thereof, such as sodium alginate. Additional excipients are especially flow conditioners and lubricants, e.g. silicic acid, talc, stearic acid or salts thereof, such as magnesium or calcium stearate, and/or polyethylene glycol, or derivatives thereof.

Dragée cores may be provided with suitable, optionally enteric, coatings, there being used, *inter alia,* concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or dragée coatings, e.g. for identification purposes or to indicate different doses of active ingredient.

Orally administrable pharmaceutical compositions are also dry-filled capsules consisting of gelatin, and also soft sealed capsules consisting of gelatin and a plasticiser, such as glycerol or sorbitol. The dry-filled capsules may contain component (a) and/or (b) in the form of granules, for example in admixture with fillers, such as corn starch, binders and/or glidants, such as talcum or magnesium stearate, and, where appropriate, stabilisers (see above for "suitable carriers"). In soft capsules, the active ingredient is preferably dissolved or suspended in suitable liquid excipients, e.g. fatty oils, ^{®}Lauroglycol (Gattefossé S.A., Saint Priest, france), ^{®}Gelucire (Gattefossé S.A., Saint Priest, France) or sesame oil, paraffin oil or liquid polyethylene glycols, such as PEG 300 or 400 (Fluka, Switzerland), or polypropylene glycols, to each of which stabilisers or detergents may also be added, or in water comprising further soluble carriers as mentioned above, such as methylcellulose or mannitol.

Other oral forms of administration are, for example, solutions or syrups prepared in customary manner that comprise component (a) and/or (b) e.g. in suspended form and in a concentration of approximately from 0.001 % to 20 %, preferably approximately 0.001% to about 2%, or in a similar concentration that provides a suitable single dose when administered, for example, in measures of 0.5 to 10 ml. Also suitable, for example, are powdered or liquid concentrates for preparing shakes, e.g. in milk. Such concentrates can also be packed in single-dose quantities.

Transdermal Delivery Systems are possible, especially with neutral active ingredients. Suitable formulations comprise, for example, about 0.0001% to about 2% by weight of component (a) and/or (b). In a preferred aspect, there are provided formulations which comprise about 2 % to 99.9999 % (or the balance to 100 %) of a short chain aliphatic alcohol. Suitable alcohols include ethanol, isopropanol, propylene glycol and glycerol. In a more preferred aspect, these formulations may additionally comprise a flux enhancer. Suitable flux enhancers include, for example, decylmethylsulfoxide, dimethylsufoxide as well as cyclic ketones, lactones, anhydrides and esters. Some of these flux enhancers also increase retention of component (a) and/or (b) and thus act to increase the concentration of it in the skin itself. For formulations for direct (local) treatment, such as topical application to the skin, it is preferred to use a flux enhancer which not only maximizes transdermal flux, but increases retention of component (a) and/or (b) in the skin. Certain cyclic ketone and lactone enhancers have been reported to increase local retention as well and, thus, comprise a preferred class of enhancers for topical administration of component (a) and/or (b). In formulations for systemic treatment, it is preferable to use a flux enhancer which maximizes flux with a minimal local retention of the active ingredient.

Suitable rectally administrable pharmaceutical compositions are e.g. suppositories that consist of a combination of the active ingredient with a suppository base. Suitable suppository bases are e.g. natural or synthetic triglycerides, paraffin hydrocarbons, polyethylene glycols or higher alkanols.

For parenteral administration there are suitable, especially, aqueous solutions of an active ingredient in water-soluble form, e.g. in the form of a water-soluble salt, in the presence or absence of salts, such as sodium chloride, and/or sugar alcohols, such as mannitol, or aqueous injection suspensions that comprise viscosity-increasing substances, e.g. sodium carboxymethylcellulose, sorbitol and/or dextran, and, where appropriate, stabilisers. Component (a) and/or (b), where appropriate together with excipients, may also be in the form of a lyophilisate and may be made into a solution prior to parenteral administration by the addition of suitable solvents.

Solutions as used e.g. for parenteral administration may also be used as infusion solutions.

Preferred formulations comprising any component (b) are those that are customary for the respective clinical use of any one or more agents belonging to that group of compounds which are known in the art.

Preferred formulations for component (a) are those mentioned in the examples. The combinations as hereinbefore described any of component (a) and/or (b), or a pharmaceutically acceptable salt thereof, may be administered prophylactically or therapeutically, preferably in an amount that is effective against the mentioned diseases, to a warm-blooded animal, e.g. man, requiring such treatment, preferably in the form of a pharmaceutical composition. The dose of any component (a) and/or (b) depends on the species of the warm-blooded animal to be treated, its body weight, its age and individual status, individual pharmacokinetic circumstances, the disease to be treated and the administration route. Preferably, for a body weight of approximately 70 kg a daily dose of from 10 mg to 2500 mg, more preferably from approximately 50 mg to approximately 2000 mg, most preferably from approximately 100 mg to approximately 1500 mg, of any one of component (a) and/or (b) is administered. Children receive a correspondingly lower dose based on their skin surface area (the skin surface area of an adult of 70 kg as reference is 1.73 m²).

The present invention can be illustrated by the following examples that are not intended to limit the scope of the present invention but serve merely as paradigmatic embodiments:

### Examples:

### Introduction

The BCR/ABL oncogenic fusion gene encodes for the hybrid bcr/abl protein that causes, due to its enhanced and constitutive tyrosine kinase activity, three different diseases: Chronic Myeloid Leukemia (CML), part of Acute Lymphoblastic Leukemia (ALL) and of Acute Myeloid Leukemia (AML).

The blocking of bcr/abl kinase activity represents an innovative and rational strategy for the treatment of CML and of the other cancers caused by this oncogenic fusion protein (Druker BJ, et al.: Effects of a selective inhibitor of the Abl tyrosine kinase on the growth of Bcr/Abl positive cells. Nat. Med. 2: 561-6, 1996).

ST1571 (formerly known as CGP57148) represents an active and relatively specific inhibitor of bcr/abl kinase activity. ST1571 blocks proliferation and induces apoptosis in BCR/ABL+ cells *in vitro;* it inhibits the growth of clonogenic bone marrow cells obtained from CML patients, and can eradicate leukemic cell growth *in vivo.* The activity of ST1571 *in vivo* is conditioned to the achievement of stable and continuous bcr/abl inhibition, which requires multiple daily administrations in the murine model which was studied (le Coutre et al., J. Natl. Cancer Inst. 91, 163-168 (1999)).

Based on this and additional information, ST1571 is now being tested in initial clinical trials in CML and in other BCR/ABL-associated diseases. Very limited information is available regarding the possible emergence of resistance to ST1571. Two cell lines have been selected to study the resistance to ST1571 *in vitro* (le Coutre P, et al., Blood 90:496a, 1997). The mechanism of resistance is unknown in one case, while it involves BCR/ABL amplification and protein overexpression in the second one. The biological relevance of these *in vitro* selected sublines remains however to be established, since the selection conditions *in vitro* are different and usually more stringent than the situation *in vivo.*

No information is available on the development and characterization of *in vivo* resistance to ST1571. In our previously described mouse model (le Coutre P, et. al., J. Natl. Cancer Inst. 91,163-168, 1999), treatment failure was noted in some animals, when treatment was delayed for one week after leukemic cell injection, and a measurable tumor mass was already present. Such a model could therefore be useful to study and characterize the possible emergence on resistance to ST1571 *in vivo.*

Here, a model for *in vivo* resistance to STI571 is established. The molecular characterization of such a model, as well as a strategy to overcome the presence of resistance is also identified and experimentally validated.

### MATERIALS AND METHODS

### ST1571

ST1571 (previously known as CGP57148B) is obtained as described in EP 0 564 409 and WO 99/03854. For the *in vitro* experiments stock solutions of this compound are prepared at 1 and at 10 mM with distilled water, filtered and stored at -20°C and then thawed before the experiment is started and used at a concentration of 0.1 - 10 µM. Preparations used for animal experiments are made daily at a concentration of 16 mg/ml and dissolved in water or in a solution of methyl cellulose 5% (Methocell, Fluka) and kept at 4°C.

### Erythromycin

For *in vitro* experiments erythromycin base (Sigma) is used. A new stock solution is prepared just before each experiment at a concentration of 20 mM and used at a concentration of 1 -100 µM. Erythromycin is dissolved in ethanol and further diluted with distilled water. For *in vivo* experiments erythromycin estolate (provided by Gist-Brocades Italy SPA, Capua, Italy) is utilized at a concentration of 35 mg/ml in a solution of methyl cellulose 5% and STI571 16 mg/ml.

### In vivo administration of STI571

Seven to 9 week-old female CD1 nu/nu mice purchased at Charles River Breedin Laboratories (Calco, Italy) are kept under standard laboratory conditions according to the guidelines of the National Cancer Institutes, Milan, Italy. This study is approved by the institutional ethics committee for laboratory animals used in experimental research. KU812 bcr/abl positive cell line is injected (50 x 10⁶ cells per animal) subcutaneously in the left flank of the animal. Oral treatment is administered through a syringe connected to a soft plastic tube introduced in esophagus (gavage). Tumor weight (TW) and total weight are monitored every 3-4 days. TW is calculated by the formula TW [mg] = (d² x D), where d and D are the shortest and longest diameters of the tumor, respectively, measured in millimeters. Treatment is started 1-15 days after leukemic cell injection.

### Cell lines

The Bcr/Abl positive human leukemia cell line KU812 is used (see Kishi K. A new leukemia cell line with Philadelphia chromosome characterized as basophil precursors. Leuk Res 9: 381-90, 1985). The KU812 line has been obtained from a CML patient in blast crisis, and is maintained in RPMI 1640 (Bio Whittaker Europe) supplemented with 10 % Fetal Calf Serum (FCS) under standard cell culture conditions.

The cell line KU812 is accessible via Deutsche Sammlung für Mikroorganismen und Zellkulturen (DSMZ), Mascheroder Weg, Braunschweig, Germany, having the accession number DSMZ No: ACC 378.

Other analogous cell lines that may be used in the tests described below are
Cell Line: BV-173 (Cell Type: human B cell precursor leukemia) DSMZ No: ACC 20;
Cell Line: K-562 (Cell Type: human chronic myeloid leukemia in blast crisis) DSMZ No: ACC 10;
Cell Line: LAMA-84 (Cell Type: human chronic myeloid leukemia in blast crisis) DSMZ No: ACC 168;
Cell Line: EM-3 (Cell Type: human chronic myeloid leukemia in blast crisis) DSMZ No: ACC 134;
Cell Line: MEG-01 (Cell Type: human chronic myeloid leukemia in megakaryocytic blast crisis) DSMZ No: ACC 364; or
Cell Line: NALM-1 (Cell Type: human chronic myeloid leukemia in blast crisis) DSMZ No: ACC 131.

### Determination of the in vitro proliferation activity (tritiated thymidine [³HtdR] uptake assay)

Two hundred microliters of each cell line (KU 812, LAMA 84), containing 104 cells, is seeded at various concentration of STI571, ranging from 0 to 10 µM in 96-well microtiter plates (Coming Costar Corp., Cambrige, MA) in six replicates. After 54 hours at 37°C, 20 µl of RPMI 1640 + 10% FCS containing tritiated thymidine (1 µCi/well) is added to each well. After an additional 18 hours, cells were harvested and transferred to a filter (Spot-on filtermat, Pharmacia Biotech Europe, Brussels, Belgium). Tritiated thymidine uptake is determinated by a 1205 betaplate liquid scintillation counter (Wallac Inc., Turku, Finland). IC₅₀ (inhibitory concentration 50) is defined as the concentration of compound producing 50% decrease of proliferation in comparison to untreated controls.

### Western blot analysis

Immunoblotting is performed as described before (Gambacorti-Passerini C et al.: Blood Cells, Molecules, and Diseases 23, 380-94, 1997). Cells are washed twice with cold phosphate-buffered saline (PBS) and subsequently lysed in 200 µl of 1 x Laemmli's buffer (50 mM Tris-HCl pH 6.8, 2% SDS, 0. 1 % bromophenol blue, 10% glycerol, 5% β-mercaptoethanol). Cell lysates, corresponding to 90,000 -150,000 cells, are boiled at 95 °C for 10 minutes, sonicated for 1 minute and analysed by SDS Gel Electrophoresis on 7.5% polyacrylamide gels. Endogenous bcr/abl, tyrosinephosphorylated bcr/abl and the endogenous actin are detected with the corresponding mouse monoclonal antibody or rabbit antiserum and then visualised by enhanced chemiluminescence detection (ECL, Amersham Corp.) using horseradish peroxidase-linked goat anti-mouse or anti-rabbit immunoglobulin G as the secondary antibody (Amersham Corp.). The monoclonal anti-abl antibody (Clone Ab-3) is purchased from Calbiochem. The monoclonal anti-phosphotyrosine antibody (clone 4G10) is purchased from Upstate Biotechnology. Rabbit polyclonal anti-actin is purchased from Sigma. Densitometric analysis is performed with an Eagle Eye 11 Photodensitometer (Stratagene) and the intensities of tyrosine-phosphorylated bcr/abl bands are normalized against the bcr/abl and actin expression levels.

### AGP determination

α₁-acidic glycoprotein (AGP) serum levels are detected by either immunodiffusion or the isoelectrofocusing method. For immunodiffusion 5.0 µl of each sample are plated into a small well of an agar plate (SRID, single radial immunodiffusion plate test, Cardiotech Services JINIC Louisville, 1 KY, USA) which contains AGP antiserum. The plate is incubated 24 hours at 37°C. The specific amount of AGP within the specimen is measured by the size of the precipitin ring and is determined by comparison to standards at 1000, 250 and 125 µg/ml, provided with each test kit. Determinations are performed in duplicates. For isoelectrofocusing an IPG (immobilized pH gradient) (Gianazza, E., Celentano, F., Ettori, C., Righetti, P. G., Immobilized pH gradients: Theory and methodology. Electrophoresis 1989, 10, 806-808) in the range of pH 2.5-5 is casted between an acidic solution containing Immobiline pK 1, 3 mM, pK 3.6, 11.83 mM, pK 9.3, 0.76 mM, and 12.9 mM Tris, and a basic solution containing Immobiline pK 3.6, 9.28 mM, pK 4.6, 9.50 mM, and pK 9.3, 16.13 mM (Amersham Pharmacia, Uppsala, Sweden). After polymerization, the gel is washed 3 times in 1 % glycerol, dried and rehydrated in 8M urea - 0.5% carrier ampholytes, pH range 2.5-5 (Pharmacia). Subsequently, 7.5 µl aliquots of sera, diluted to 25 µl with 2% 2-mercaptoethanol, are loaded near the cathode. After an overnight run at 55 V/cm, the samples are focused for 90 min at 165 V/cm. The protein pattern is stained with Coomassie.

### Estimation of binding parameters

Plasma or purified human AGP (Sigma) or Albumin (diluted in PBS) are incubated with various STI571 concentrations at room temperature for 30 min. STI571 concentrations are determined by HPLC, with a lower limit of detection of 0.1 µM. Free STI571 is determined by ultrafiltration with a cut off at 30 KD. Modified Scatchard plots are constructed. The Association Constant (Ka) is calculated as previously described (Fuse E, Tanii H, Kurata N et al., Cancer Res., 58, 3248-53, 1998).

### Statistical analysis

Statistical analysis is performed with Fisher exact test or T Student using the Prism analysis program. For survival analysis, data are compared by the logrank test. P values <0.05 are considered statistically significant and are derived from two-sided statistical tests.

### Example 1: STI571 efficacy is related to the initial tumor load

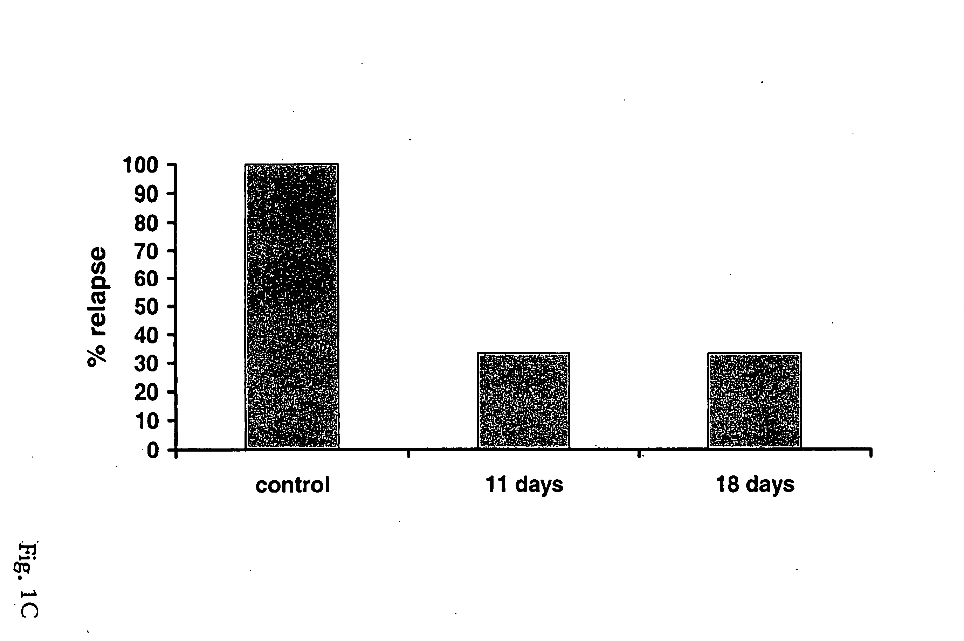

Nude mice are injected s.c. with 50 millions KU812 cells. Treatment is initiated after 1, 8 and 15 days respectively (groups I to III), in the presence of approximately, 50, 300 millions and 1 billion leukemic cells. Although tumor regression is observed in all groups, cures are obtained only in the first two groups. Fig. 1A shows the results of a representative experiment. While all animals in group I are reproducibly cured, mice in group II develop between 33% and 40% relapses; no cure is ever observed in group III. Relapses usually develop 1 to 3 weeks after treatment discontinuation. Fig. 1B presents the combined results from 3 different experiments. A clear relationship between the amount of tumor present at the beginning of treatment and the outcome of the therapy is evident. A possible explanation for these results could reside in the insufficient length of STI571 administration in group II and III. To test this hypothesis mice in group II are treated for 11 or 18 days. The result of one representative experiment is shown in Fig. 1C and indicates that increasing the duration of treatment does not ameliorate the cure rate. These results indicate that in this model the treatment with STI571 can cure animals only if the tumor is eradicated in the first 11 days of treatment. If this does not happen, cure cannot be achieved, even with longer exposure to the compound. In other words: some type of resistance has emerged.

### Example 2: Relapsed tumors show in vivo resistance but retain in vitro sensitivity to STI571

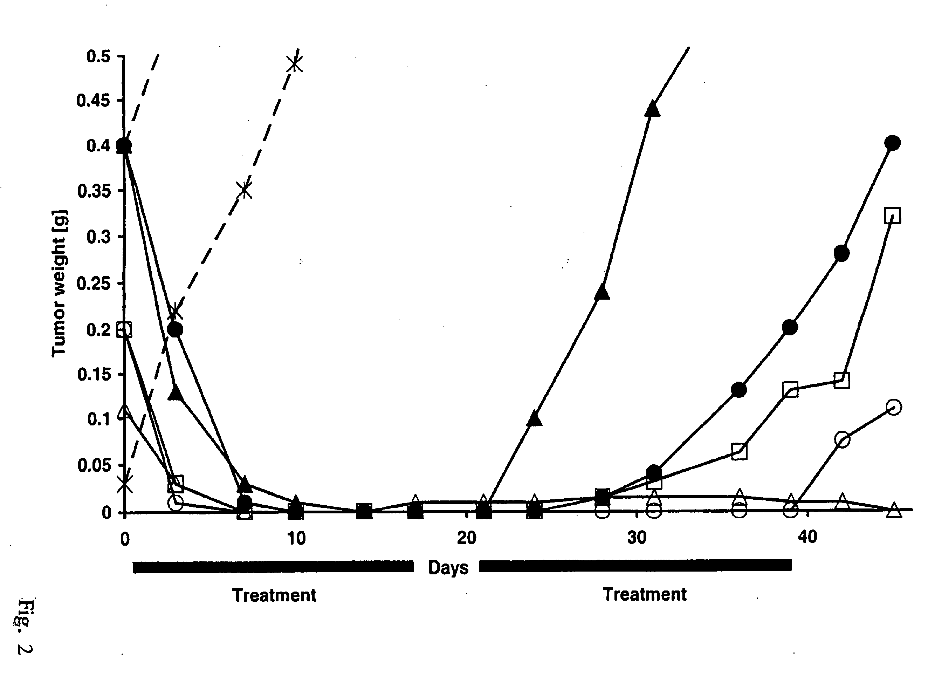

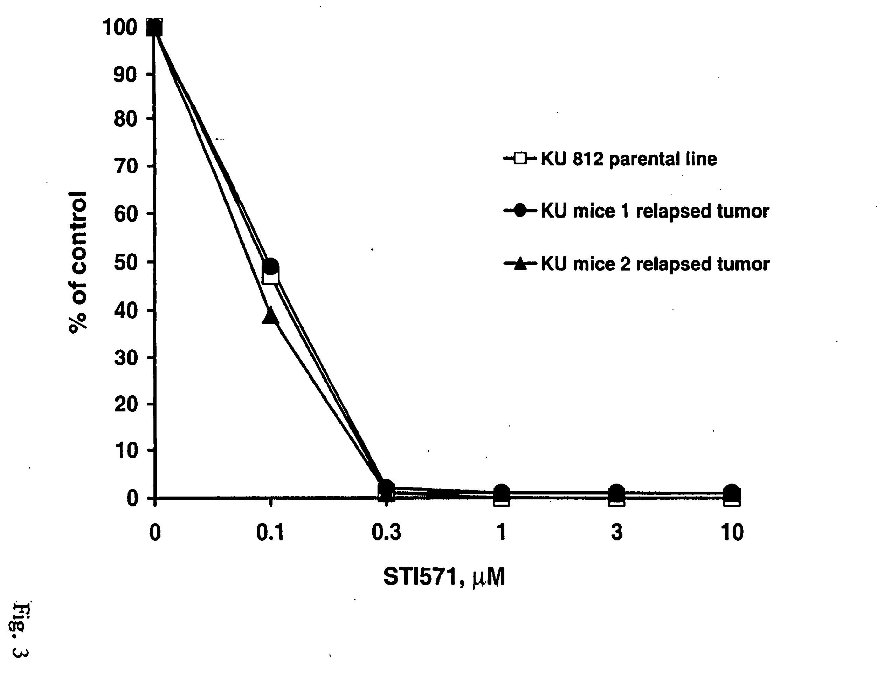

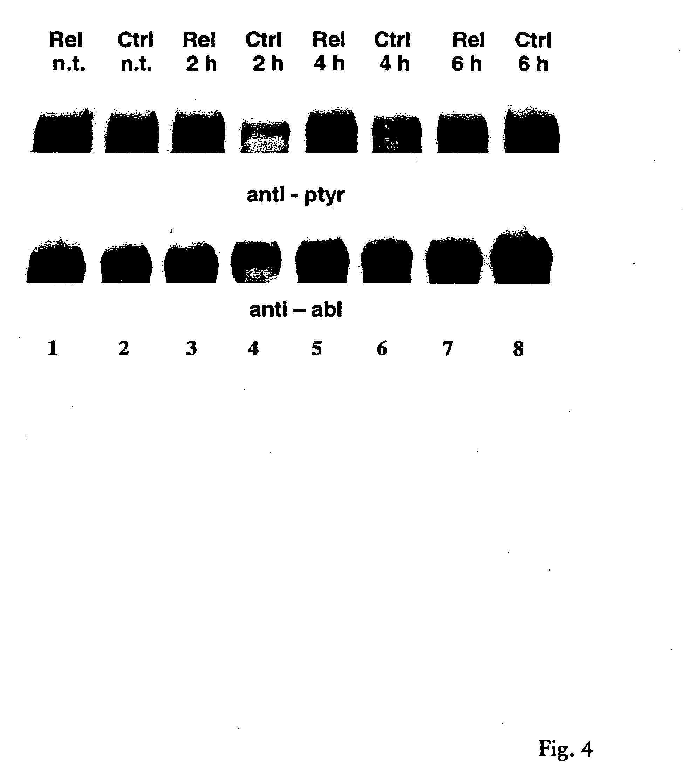

Animals presenting recurrent tumors are retreated with the same STI571 schedule (11 day regimen). Treatment starts as soon as tumors become again measurable. Fig. 2 shows one representative experiment. It is evident that relapsed tumors respond poorly to the new treatment, and eventually start to grow similarly to tumors in untreated animals (dashed lines). Although leukemic cells are resistant *in vivo* to STI571, their intrinsic sensitivity to STI571 is not known. To investigate this issue, tumors are excised from resistant animals, cell suspensions are obtained and cells are placed in culture and tested for *in vitro* sensitivity to STI571 within 24-48 hours, as previously described (le Coutre P, et al., J. Natl. Cancer Inst. 91,163-168, 1999). Fig. 3 presents the results obtained from two such tumors. It is evident that the leukemic cells obtained from resistant animals retain their sensitivity to STI571, as their IC₅₀ does not differ from that of the parental KU812 cell line. These results lead us to evaluate whether the kinase activity of the bcr/abl protein is still inhibited by STI571 administration. To this aim molecular pharmacokinetics experiments (described in: le Coutre P, et al., J. Natl. Cancer Inst. 91,163-168, 1999) are performed, to investigate the degree and duration of *in vivo* Bcr/Abl inhibition in animals that are not pretreated or in mice that relapsed after the initial treatment and were resistant to a second cycle of STI571 administration. Tumor bearing mice are treated acutely and killed at 2 and 4 hours. The levels of Bcr/Abl kinase activity (measured as autophosphorylation) obtained in a representative experiment are shown in Fig. 4. While non-pretreated mice show the previously reported inhibition in bcr/abl phosphorylation at both 2 and 4 hours (lanes 4 and 6), relapsed animals resistant to STI571 are not inhibited by the treatment (lanes 3 and 5). These experiments indicate that relapsed mice are resistant to further treatment and that inhibition of bcr/abl kinase activity was not achieved in this situation. Leukemic cells however retain their *in vitro* sensitivity to STI571.

### Example 3: STI571 plasma levels in relapsed mice

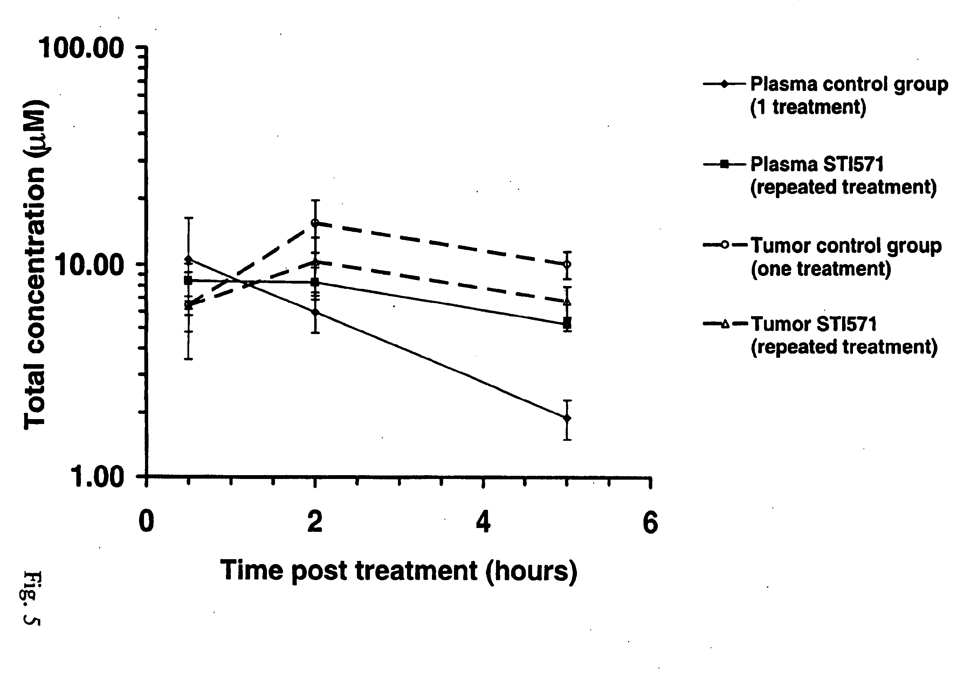

A possible explanation for the above reported results could reside in an increased metabolism of STI571 in pretreated animals, with resulting lower STI571 plasma levels. To investigate this issue, tumor bearing mice, either pretreated with STI571 or not (controls), are killed at 0.5, 2.0 and 5.0 hours after an acute treatment with STI571 and the plasma STI571 total concentrations determined by HPLC. The results are presented in Fig. 5. While control and pre-treated animals reach similar plasma levels at 30', STI571 levels decrease more quickly in control animals, compared with pretreated mice (p<0.01). Intra tumor concentrations show an opposite pattern, tumors present in pretreated animals contain lower STI571 concentrations at all time points, this phenomenon reaching statistical significance at the 5 hour time point. These results do not confirm our hypothesis, and even show that resistant animals maintain higher STI571 concentrations in their blood for a longer time. These data, among other alternatives, could instead be compatible with the presence, in the blood of resistant mice, of a "factor" able to bind and decrease the clearance and biological activity of STI571.

### Example 4: α₁-Acidic Glycoprotein (AGP) binds STI571 and inhibits its effects

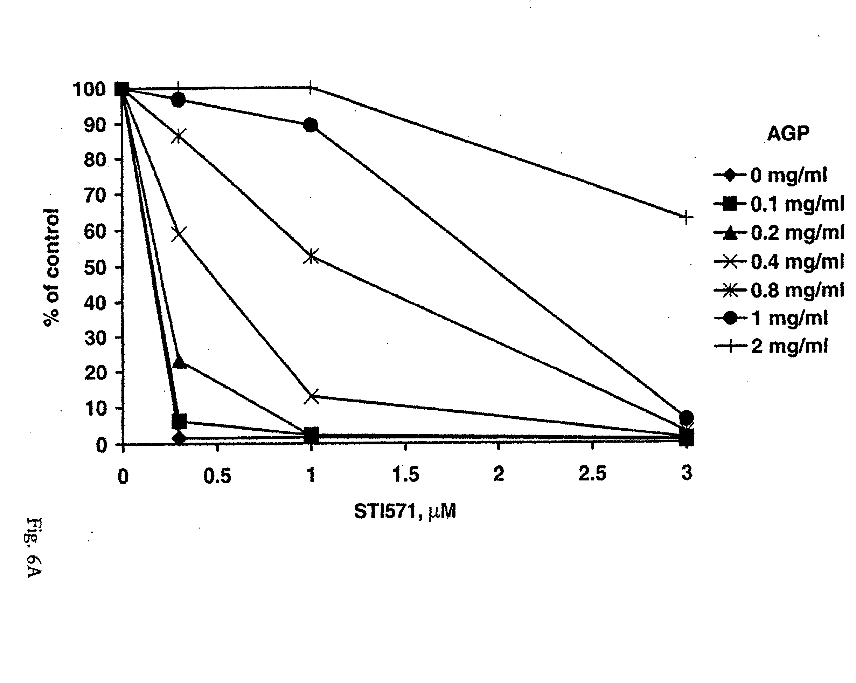

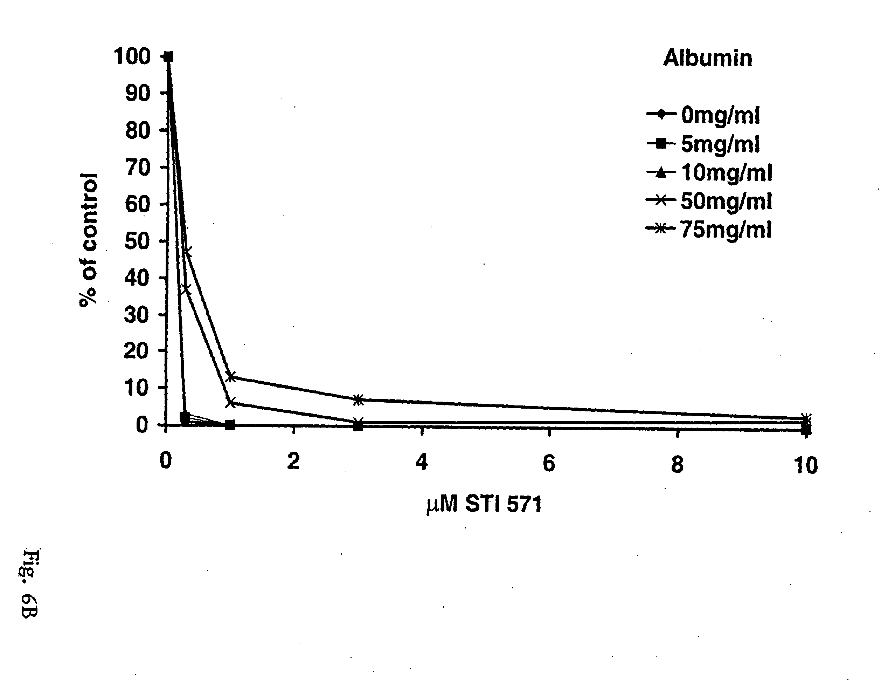

Two plasma proteins that can bind drugs are albumin and AGP. AGP preferentially binds basic molecules (Kremer et al., Drug binding to human α1-acidic glycoprotein in health and disease, Pharmacological Reviews 40, 1-47, 1988). KU812 cells are used *in vitro* to assess the effect of AGP on the biological activity of STI571. Since murine AGP is unavailable in quantities sufficient for this type of experiments (Fuse E, et al., Cancer Res. 58, 3248-53, 1998), human AGP is used. The results of one representative experiment are presented in Fig. 6A. It is evident that AGP inhibits the activity of STI571 (measured as IC₅₀); this effect is proportional to the concentration of AGP. The IC₅₀ increases from the usual 0.05 µM in the absence of AGP (Gambacorti-Passerini C, et al., Blood Cells, Molecules, and Diseases 23, 380-94, 1997), to over 3.0 µM at an AGP concentration of 2 mg/ml. In a separate experiment the IC₅₀ at 2.0 mg/ml AGP is calculated at 4.5 µM (data not shown). Albumin does not substantially increase the IC₅₀ for STI571, even at 50 mg/ml (Fig. 6B). Therefore, AGP but not albumin can increase the IC₅₀ for STI571 up to values 90 times higher than in controls.

Since an inhibitory effect of AGP is noted, the Association Constant (Ka) of STI571 for AGP and for albumin are calculated. To this aim a fixed amount of AGP (5 µM) is incubated with different STI571 concentrations and the amount of unbound drug (free fraction) is evaluated by ultrafiltration. Scatchard plot curves are obtained for both AGP and albumin. In the case of AGP a value of 8.7 liters/moles is found, which is approximately 60 times higher than the Ka calculated for albumin (0.15). These results indicate that although both albumin and AGP can bind STI571, the latter binds STI571 with much higher affinity and, as result, inhibits the biological activity of STI571.

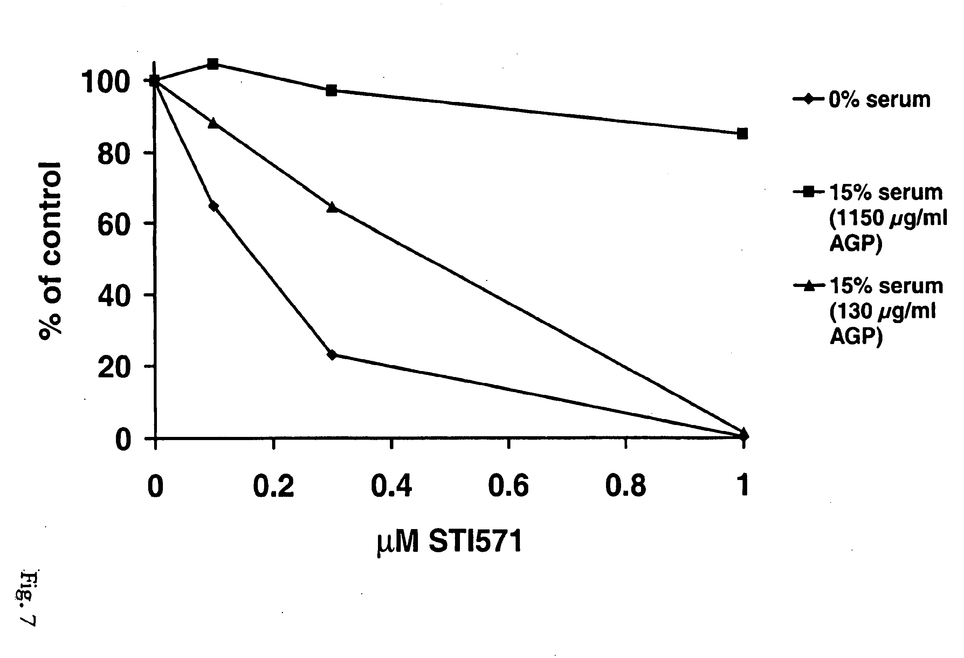

To confirm the above-mentioned results, KU812 cells are challenged *in vitro* with sera containing different amount of AGP. Fig. 7 presents one representative experiment in which two sera containing 130 µg/ml AGP (triangles) and 1150 µg/ml AGP (squares), respectively, are added (at a final concentration of 15%) to KU812 cultures (control: 0% serum; diamonds). It is evident that the inhibition of STI571 activity is associated with the respective AGP content of each serum sample. The change in the percentage of serum present in the culture produces a proportional increase or decrease in its inhibitory activity (not shown). It is concluded that AGP can bind STI571, this binding has important biological consequences and blocks the ability of STI571 to inhibit the enzymatic activity of the Bcr/Abl kinase.

### Example 5: Relationship among AGP serum levels, tumor load and STI571 treatment

The results previously presented indicate that AGP, an inducible plasma protein, potently inhibits the activity of STI571 *in vitro.* The plasma levels of AGP are determined in nude mice at various stages of disease by immunodiffusion, to assess whether those values can be associated to the *in vivo* sensitivity of KU812 leukemic cells to STI571 treatment. Fig. 8A presents the AGP values in mice in various stages of disease. Basal AGP values in mice are very low (96 ± 21 µg/ml). AGP concentrations rise proportionally to the tumor load present. Mice with a tumor load of approximately 200 mg (corresponding approximately to 200 million leukemic cells) show a fourfold increase in AGP values (383 ± 131 µg/ml), while mice with 0.8 - 1 g of tumors show AGP values in excess of 1 mg/ml (1580 ± 234 µg/ml). Animals with measurable tumors that are cured showed a progressive decrease in AGP concentrations and return to normal levels in 4-8 weeks. Experiments with purified AGP indicate that the variations in AGP concentrations observed between normal mice and animals bearing large tumors cause a change in AGP-bound STI571 fraction from 42% to over 99% (not shown). Interestingly STI571 treatment (160 mg/kg p.o. for 11 days) also produces lower but statistically significant increase of AGP values (213 ± 43 µg/ml). The increased AGP levels in tumor-bearing mice are also evidenced by isoelectrofocusing (Fig. 8B). These results, taken together, show that tumor load (and to a minor extent STI571 pretreatment) induces the synthesis of AGP, a plasma protein that, in turn, can bind and inactivate STI571.

### Example 6: Erythromycin, a binder of AGP, can relieve the AGP-mediated block of STI571 activity in vitro

(a) Several drugs are known to bind AGP, including erythromycin (Kremer et al., Pharmacological reviews 40, 1-47,1988). If the mechanism by which AGP blocks STI571 is mediated by the binding of AGP to STI571, then a third molecule able to bind AGP could compete with STI571 for AGP binding, thus rendering more STI571 available for biological activity. To validate such a hypothesis, erythromycin is added at 5 to 30 µM to KU812 cultures containing STI571 (1 µM). AGP (1 mg/ml), or both STI571 (1 µM) and AGP (1mg/ml). The results of one representative experiment are presented in Fig. 9 (STI alone: squares; AGP alone: triangles; STI and AGP: diamonds). Erythromycin restores sensitivity to STI571 with a clear dose response relationship. Erythromycin does not modify the IC₅₀ for STI571 in the absence of AGP, thus excluding a direct effect on STI571 anti-leukemic effect (not shown).
(b) The effects of erythromycin are also assessed on the STI571-mediated block of bcr/abl kinase activity (Fig. 10). KU812 cells are treated *in vitro* with STI571, AGP and erythromycin, incubated at 37°C for 1 hour and lysed; the amount of kinase activity is then evaluated using an anti-phosphotyrosine antibody. Fig. 10 shows that AGP inhibits the activity of STI571. STI571 activity can be restored by the addition of erythromycin.

Experiments (a) and (b) provide evidence in two different assays, that erythromycin can restore STI571 sensitivity *in vitro.*

### Example 7: In vivo effects of erythromycin administration and of STI571 pretreatment

(a) Having demonstrated the inhibitory effect of AGP and its reversal by erythromycin in *vitro,* we experimentally validate the *in vivo* modulation of AGP values or of its binding ability. Mice are injected with KU812 cells and treatment is started 11 days later in the presence of an approximate tumor load of 400 mg. In this situation few or no cures are expected from a standard STI571 treatment. Mice are treated with STI571 (160 mg/kg p.o. every 8 hours) alone or in combination with erythromycin estolate (350 mg/kg every 8 hours) for 18 days. The estolate formulation of erythromycin is chosen since its good oral bioavailability in mice was previously demonstrated, the selected dose being expected to produce peak concentrations higher than 20 µM. As exemplified in Fig. 11A, the combined treatment produces a significantly higher tumor reduction at day 6, and from day 16 onward. It is important to note that while tumor regression is progressive in mice receiving the combined treatment, some tumors start to regrow during the last days of treatment in the group treated with STI571 only. The effect of adding erythromycin to STI571 is even more evident when the cure rates in the two groups are compared (Fig. 11 B). In the series receiving STI571 alone, 5/15 animals show disappearance of the tumor. However 4 animals relapsed between day 25 and 40, with only 1/13 animals being cured at day 120 (last day of follow up). In the group that received the combined erythromycin/STI571 therapy, 14/15 mice become tumor free, and only 1 animal relapses, at day 30. Therefore, 10/12 animals are cured by the combined treatment; this value is significantly different (p<0.01) from the one (1/13) obtained in the STI571 only group. Control groups receiving erythromycin alone do not show any evidence of tumor regression (not shown).
(b) The biological effects of STI571 pretreatment are also evaluated. Mice are pretreated or not with STI571 for 14 days, injected with KU812 cells and then treated with STI571 (160 mg/kg every 8 hours for 11 days), starting 24 hours after leukemic cells injection. Under these circumstances, 100% animals are expected to be cured by STI571. The results are present in Fig. 12. In the control group (non pretreated mice) 0/14 animals developed tumor growth. In the group of pretreated mice, tumor growth occurs in 7/14 animals (p<0.05), indicating that the AGP increase associated with previous STI571 treatment can also produce a significant biological effect. These results, taken together, support experimental evidence to the negative effects of AGP on the therapeutic activity of STI571; they also suggest and provide partial experimental validation to a strategy aimed at bypassing *in vivo* resistance mediated by AGP, using molecules able to bind to AGP in competition with STI571.

### Example 8: Tablets with 4-[(4-methyl-1-piperazin-1-ylmethyl)-N-[4-methyl-3-[[4-(3-pyridinyl)-2-pyrimidinyl]amino]-phenyl]benzamide methanesulfonate. β-crystal form

Tablets containing 100 mg of the active substance named in the title are usually prepared in the following composition:

| Composition | |
|---|---|
| Active ingredient | 100 mg |
| Crystalline lactose | 240 mg |
| Avicel | 80 mg |
| PVPPXL | 20 mg |
| Aerosil | 2 mg |
| Magnesium stearate | 5 mg |
| | 447 mg |

Preparation: The active substance is mixed with carrier materials and compressed on a tableting machine (Korsch EKO, punch diameter 10 mm).
Avicel is microcrystalline cellulose (FMC, Philadelphia, USA).
PVPPXL is polyvinylpolypyrrolidone, cross-linked (BASF, Germany).
Aerosil is silicon dioxide (Degussa, Germany).

### Example 9: Capsules with 4-[(4-methyl-1-piperazin-1-ylmethyl)-N-[4-methyl-3-[[4-(3-pyridinyl)-2-pyrimidinyl]amino]phenyl]benzamide methanesulfonate. β-crystal form

Capsules containing 100 mg of the compound named in the title as active substance are usually prepared in the following composition:

| Composition | |
|---|---|
| Active ingredient | 100 mg |
| Avicel | 200 mg |
| PVPPXL | 15 mg |
| Aerosil | 2 mg |
| Magnesium stearate | 1.5 mg |
| | 318.5 mg |

The capsules are prepared by mixing the components and filling the mixture into hard gelatin capsules, size 1. PVPPXL = Crospovidone XL (see Example 10).

### Example 10: Capsules with 4-[(4-methyl-1-piperazin-1-ylmethyl)-N-[4-methyl-3-[[4-(3-pyridinyl)-2-pyrimidinyl]amino]phenyl]benzamide methanesulfonate. β-crystal form

Crospovidone XL: Cross-linked povidone = water insoluble cross-linked homopolymer of N-vinyl-2-pyrrolidone
Aerosil 200: pure silica gel (surface area according to BET 200 ± 25 m²/g. mean grain size 12 nm).
Avicel: microcrystalline cellulose.

The composition of the capsule fill for the 100, 50 and 25mg capsules is identical. The different dosage strengths are obtained by varying the capsule fill weight only. The intended capsule sizes are 100mg size 1, 50mg size 3 and 25mg size 4.

**Table 1: Composition for capsules (Quantities used per each batch in [kg])**

| **Excipient** | **Percent** | **100mg** | **50mg** | **25mg** |
|---|---|---|---|---|
| 4-(4-methylpiperazin-1-ylmethyl)-N-[4-methyl-3-(4-pyridin-3-yl)pyrimidin-2-ylamino)phenyl]-benzamide methansulfonate salt in the β-crystal form | 51.96 | 119.50 | 59.75 | 29.875 |
| Avicel PH 102 | 40.00 | 92.00 | 46.00 | 23.000 |
| Crospovidone XL | 6.52 | 15.00 | 7.50 | 3.750 |
| Aerosil 200 | 0.87 | 2.00 | 1.00 | 0.500 |
| Magnesium Stearate | 0.65 | 1.50 | 0.75 | 0.375 |
| **Total** | **100.00** | **230.00** | **115.00** | **57.50** |
| Capsule size | | 1 | 3 | 4 |

**Table 2:**

| **Ingredient** | **Percent** | **100mg** | **50mg** |
|---|---|---|---|
| | **QUANTITY PER BATCH (%)** | **QUANTITY PER BATCH (kg)** | **QUANTITY PER BATCH (kg)** |
| 4-(4-methylpiperazin-1-ylmethyl)-N-[4-methyl-3-(4-pyridin-3-yl)pyrimidin-2-ylamino)phenyl]benzamide methansulfonate salt in the β-crystal form | 51.96 | 18.224 | 9.112 |
| Cellulose MK GR | 40.00 | 14.030 | 7.015 |
| Crospovidone XL | 6.52 | 2.288 | 1.144 |
| Aerosil 200 | 0.87 | 0.305 | 0.153 |
| Magnesium Stearate | 0.65 | 0.228 | 0.114 |
| **Total** | **100.00** | **35.075** | **17.538** |
| Capsule size | | 1 | 3 |
| **Amount of capsules** | | **152'500** | **152'500** |

### Example 11: Composition of typical Erythromycin formulation

Here any standard formulation known for erythromycin may be used.

An example for a formulation with erythromycin estolate is given in Table 3.

**Table 3: (Quantities used per each batch in [kg])**

| **Excipient** | **Percent** | **100mg** | **50mg** | **25mg** |
|---|---|---|---|---|
| erythromycin estolate | 51. 96 | 119.50 | 59.75 | 29.875 |
| Avicel PH 102 | 40.00 | 92.00 | 46.00 | 23.000 |
| Crospovidone XL | 6.52 | 15.00 | 7.50 | 3.750 |
| Aerosil 200 | 0.87 | 2.00 | 1.00 | 0.500 |
| Magnesium Stearate | 0.65 | 1.50 | 0.75 | 0.375 |
| **Total** | **100.00** | **230.00** | **115.00** | **57.50** |

### Example 12: Possible combined composition with both Erythromycin and STI571

**Table 4: (Quantities used per each batch in [kg])**

| **Excipient** | **Percent** | **100mg** | **50mg** | **25mg** |
|---|---|---|---|---|
| 4-(4-methylpiperazin-1-ylmethyl)-N-[4-methyl-3-(4-pyridin-3-yl)pyrimidin-2-ylamino)phenyl]-benzamide methansulfonate salt in the β-crystal form | 25.98 | 59.75 | 29.875 | 14.9375 |
| erythromycin estolate | 25.98 | 59.75 | 29.875 | 14.9375 |
| Avicel PH 102 | 40.00 | 92.00 | 46.00 | 23.000 |
| Crospovidone XL | 6.52 | 15.00 | 7.50 | 3.750 |
| Aerosil 200 | 0.87 | 2.00 | 1.00 | 0.500 |
| Magnesium Stearate | 0.65 | 1.50 | 0.75 | 0.375 |
| **Total** | **100.00** | **230.00** | **115.00** | **57.50** |

## Claims

1. The use of a combination of
(a) at least one abl-, PDGF-R-(platelet-derived growth factor receptor) and/or Kit receptor-tyrosine kinase inhibitor and
(b) at least one organic compound capable of binding to α₁-acidic glycoprotein,
wherein any component (a) and/or (b) can also be present in the form of a pharmaceutically acceptable salt, if at least one salt-forming group is present, for producing a pharmaceutical preparation for use as compositions against a proliferative disease that can be treated by administration of an abl-, PDGF-R- and/or Kit receptor-tyrosine kinase inhibitor, which is or, during treatment with 4-(4-methylpiperazin-1-ylmethyl)-N-[4-methyl-3-(4-pyridin-3-yl)pyrimidin-2-ylamino)phenyl]benzamide, is becoming or has become completely or partially resistant to such treatment.

2. A combination preparation comprising (a) at least one abl-, PDGF-R- (platelet-derived growth factor receptor) and/or Kit receptor-tyrosine kinase inhibitor and (b) at least one organic compound capable of binding to α₁-acidic glycoprotein (AGP); or pharmaceutically acceptable salts of any component (a), (b) or (a) and (b) if at least one salt-forming group is present, and a pharmaceutically acceptable carrier, wherein the abl-, PDGF-Receptor-and/or Kit receptor-tyrosine kinase inhibitor (a) is selected from
(i) 1-(4-chloro-anilino)-4-(4-pyridyl-methyl)-phtalazine
and
(ii) an N-phenyl-2-pyrimidine-amine derivative of formula I 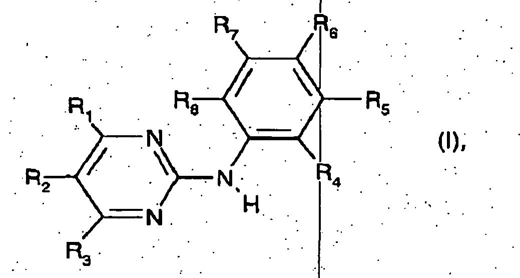
wherein
R₁ is pyrazinyl, 1-methyl-1H-pyrrolyl, amino- or amino-lower alkyl-substituted phenyl wherein the amino group in each case is free, alkylated or acylated, 1 H-indolyl or 1 H-imidazolyl bonded at a five-membered ring carbon atom, or unsubstituted or lower alkyl-substituted pyridyl bonded at a ring carbon atom and unsubstituted or substituted at the nitrogen atom by oxygen,
R₂ and R₃ are each independently of the other hydrogen or lower alkyl,
one or two of the radicals R₄, R₅, R₆, R₇ and R₈ are each nitro, fluoro-substituted lower alkoxy or a radical of formula II
-N(R₉)-C(=X)-(Y)ₙ-R₁₀ (II),
wherein
**R₉** is hydrogen or lower alkyl,
X is oxo, thio, imino, N-lower alkyl-imino, hydroximino or O-lower alkyl-hydroximino,
Y is oxygen or the group NH,
n is 0 or 1 and
R₁₀ is an aliphatic radical having at least 5 carbon atoms, or an aromatic, aromatic-aliphatic, cycloaliphatic, cycloaliphatic-aliphatic, heterocyclic or heterocyclic-aliphatic radical, and the remaining radicals R₄, R₅, R₆, R₇ and R₈ are each independently of the others hydrogen, lower alkyl that is unsubstituted or substituted by free or alkylated amino, piperazinyl, piperidinyl, pyrrolidinyl or by morpholinyl, or lower alkanoyl; trifluoromethyl, free, etherified or esterifed hydroxy, free, alkylated or acylated amino or free or esterified carboxy, or
a salt of such compounds having at least one salt-forming group;
and the organic compound capable of binding to α₁-acidic glycoprotein (AGP) (b) is selected from :
Nicergoline, Prazosin, Alfentanil, Ketamine, Ethidocaine, Fentanil, Meperidine, Methadone, Phenylbutazone, Bupivacaine, Etidocaine, Phencyclidine, Lidocaine, Phencyclidin, Aprindine, Disopyramide, Quinidine, Verapamil, Erythromycin, Acenocoumarol, Dipyridamole, Ticlopidine, Warfarin, Phenytoin, Carbamazepine, Naproxen, Alprenolol, Metoprolol, Oxprenolol, Pindolol, Propranolol, Timolol, Progesterone, Cortexone, Testosteron, Estradiol, Prednisolone, Metocurine, d-Tubocurarine, Amitriptyline, Chlorpromazine, Cyclazindol, Desmethylimipramine, Diazepam, Doxepine, Flurazepam, Fluphenazine, Haloperidol, Imipramine, Loxapine, Mianserin, Nortriptyline, Norzimelidine, Perazine, Perphenazine, Phenobarbital, Phenothiazine derivatives, Promazine, Acepromazine, Protipendyl, Thioridazine, Thiothixene, Triazolam, Trifluoperazine, Zimelidine, Vitamin B₁₂, folic acids 1,8-Anilino-naphthalene sulfonate, Aminopyrine, Amoxapine, Bupropion, Maprotiline, Nomifensine, Trazodone, Ritodrine, Doxazosin, Trimazosin, Binedalin, Amsacrine, Apazone, Ciclazindol, Indomethacin, Probenecid, Retinoic Acid, Sulfinpyrazone, Tolmetin, Benoxaprofen, Heparin, Sufentanil, Lofentanil, Metoclopramide, Nicardipine, Pirmenol, mifepristone, Aprindil, Auramine O, Bepridil, Desipramine, Desmethylclomipramine, Moxaprindine, Quinine, Lorcainide, Prothipendyl, Protriptyline, Trihexyphenidyl, Biperiden, Methaqualone, Diphenhydramine, Glutethimide, Chlordiazepoxid, L-Tryptophane, Mepivacaine, Levomethadone, Opipramol, Trifluopromazine, Trimipramine, tris-butoxyethyl phosphate, staurosporine, N-benzoyl-staurosporine and 7-hydroxy staurosporine;
wherein any component (a) and/or (b) can also be present in the form of a pharmaceutically acceptable salt, if at least one salt-forming group is present.

3. A combination preparation according to claim 2, wherein (a) at least one abl-, PDGF-Receptor- and/or Kit receptor-tyrosine kinase inhibitor selected from the group consisting of 1-(4-chloro-anilino)-4-(4-pyridyl-methyl)-phthalazine and 4-(4-methylpiperazin-1-ylmethyl)-N-[4-methyl-3-(4-pyridin-3-yl)pyrimidin-2-ylamino)phenyl]benzamide; or a pharmaceutically acceptable salt of any one or more of these compounds; and
(b) at least one compound capable of binding to α₁-acidic glycoprotein selected from the group consisting of an antibiotic, staurosporine, N-benzoyl-staurosporine and 7-hydroxy-staurosporine, or a pharmaceutically acceptable salt thereof, are combined.

4. A combination preparation according to claim 2, wherein (a) the abl-, PDGF-Receptor-and/or Kit receptor-tyrosine kinase inhibitor is 4-(4-methylpiperazin-1-ylmethyl)-N-[4-methyl-3-(4-pyridin-3-yl)pyrimidin-2-ylamino)phenyl]benzamide, or a pharmaceutically acceptable salt thereof, and (b) the compound capable of binding to α₁-acidic glycoprotein is Erythromycin, or a pharmaceutically acceptable salt thereof.

5. A product which comprises
(a) at least one abl-, PDGF-R- (platelet-derived growth factor receptor) and/or Kit receptor-tyrosine kinase inhibitor and
(b) at least one organic compound capable of binding to α₁-acidic glycoprotein,
wherein any component (a) and/or (b) can also be present in the form of a pharmaceutically acceptable salt, if at least one salt-forming group is present,
in the presence or absence of one or more pharmaceutically acceptable carrier materials, as a combination preparation for simultaneous or chronologically staggered use within a period of time which is small enough for the active compounds both of component (a) and of component (b) to enhance antiproliferative activity of compound (a) against proliferating cells, especially in a patient, for treating a proliferative disease which responds to such a compound, wherein the abl-, PDGF-Receptor- and/or Kit receptor-tyrosine kinase inhibitor is selected from the group consisting of
(i) 1-(4-chloro-anilino)-4-(4-pyridyl-methyl)-phthalaxine and
(ii) an N-phenyl-2-pyrimidine-amine derivative of formula I 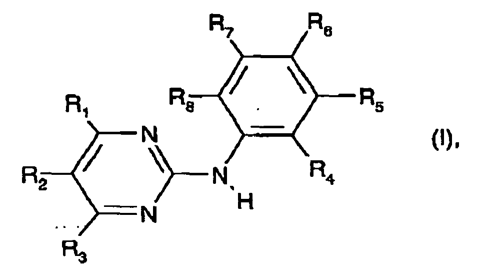
wherein
R₁ is pyrazinyl, 1-methyl-1H-pyrrolyl, amino- or amino-lower alkyl-substituted phenyl wherein the amino group in each case is free, alkylated or acylated, 1H-indolyl or 1 H-imidazolyl bonded at a five-membered ring carbon atom, or unsubstituted or lower alkyl-substituted pyridyl bonded at a ring carbon atom and unsubstituted or substituted at the nitrogen atom by oxygen,
R₂ and R₃ are each independently of the other hydrogen or lower alkyl,
one or two of the radicals R₄, F₅, R₆, R₇ and R₈ are each nitro, fluoro-substituted lower alkoxy or a radical of formula II
-N(R₉)-C(=X)-(Y)ₙ-F₁₀ (II),
wherein
R₉ is hydrogen or lower alkyl,
X is oxo, thio, imino, N-lower alkyl-imino, hydroximino or O-lower alkyl-hydroximino,
Y is oxygen or the group NH,
n is 0 or 1 and
R₁₀ is an aliphatic radical having at least 5 carbon atoms, or an aromatic, aromatic-aliphatic, cycloaliphatic, cycloaliphatic-aliphatic, heterocyclic or heterocyclic-aliphatic radical, and the remaining radicals R₄, R₅, R₆, R₇ and R₈ are each independently of the others hydrogen, lower alkyl that is unsubstituted or substituted by free or alkylated amino, piperazinyl, piperidinyl, pyrrolidinyl or by morpholinyl, or lower alkanoyl, trifluoromethyl, free, etherified or esterifed hydroxy, free, alkylated or acylated amino or free or esterified carboxy, or
a salt of such compounds having at least one salt-forming group;
and the organic compound capable of binding to α₁-acidic glycoprotein (AGP) (b) is selected from.
Nicergoline, Prazosin, Alfentanil, Ketamine, Ethidocaine, Fentanil, Meperidine, Methadone, Phenylbutazone, Bupivacaine, Etidocaine, Phencyclidine, Lidocaine, Phencyclidin, Aprindine, Disopyramide, Quinidine, Verapamil, Erythromycin, Acenocoumarol, Dipyridamole, Ticlopidine, Warfarin, Phenytoin, Carbamazepine, Naproxen, Alprenolol, Metoprolol, Oxpronolol, Pindolol, Propranolol, Timolol, Progesterone, Cortexone, Testosteron, Estradiol, Prednisolone, Metocurine, d-Tubocurarine, Amitriptyline, Chlorpromazine, Cyclazindol, Desmethylimipramine, Diazepam, Doxepine, Flurazepam, Fluphenazine, Haloperidol, Imipramine, Loxapine, Mianserin, Nortriptyline, Norzimelidine, Perazine, Perphenazine, Phenobarbital, Phenothiazine derivatives, Promazine, Acepromazine, Protipendyl, Thioridazine, Thiothixene, Triazolam, Trifluoperazine, Zimelidine, Vitamin B₁₂, folic acid, 1,8-Anilino-naphthalene sulfonate, Aminopyrine, Amoxapine, Bupropion, Maprotiline Nomifensine, Trazodone, Ritodrine, Doxazosin, Trimazosin, Binedalin, Amsacrine, Apazone, Ciclazindol, Indomethacin, Probenecid, Retinoic Acid, Sulfinpyrazone, Tolmetin, Benoxaprofen, Heparin, Sufentanil. Lofentanil, Metoclopramide. Nicardipine, Pirmenol, mifepristone, Aprindil, Auramine O, Bepridil, Desipramine, Desmethylclomipramine Moxaprindine, Quinine, Lorcainide, Prothipendyl, Protriptyline, Trihexyphenidyl, Biperiden, Methaqualone, Diphenhydramine, Glutethimide, Chlordiazepoxid, L-Tryptophane, Mepivacaine, Levomethadone, Opipramol, Trifluopromazine, Trimipramine, tris-butoxyethyl phosphate, staurosporine, N-benzoyl-staurosporine and 7-hydroxy staurosporine;
wherein any component (a) and/or (b) can also be present in the form of a pharmaceutically acceptable salt, if at least one salt-forming group is present.

6. The product according to claim 5, wherein (a) the abl-, PDGF-Receptor- and/or Kit receptor-tyrosine kinase inhibitor is selected from 1-(4-chloro-anilino)-4-( 4-pyridyl-methyl)-phthalazine and 4-(4-methylpiperazin-1-ylmethyl)-N-[4-methyl-3-(4-pyridin-3-yl)pyrimidin-2-ylamino)phenyl]benzamide; or a pharmaceutically acceptable salt of any one or more of these compounds; and
(b) the compound capable of binding to α₁-acidic glycoprotein is selected from an antibiotic, staurosporine, N-benzoyl-staurosporine and 7-hydroxy-staurosporine, or a pharmaceutically acceptable salt of any one or more of these compounds.

7. The product according to claim 5, wherein (a) the abl-, PDGF-Receptor- and/or Kit receptor-tyrosine kinase inhibitor is 4-(4-methylpiperazin-1-ylmethyl)-N-[4-methyl-3-(4-pyridin-3-yl)pyrimidin-2-ylamino)phenyl]benzamide, or a pharmaceutically acceptable salt thereof, and (b) the compound capable of binding to α₁-acidic glycoprotein is Erythromycin, or a pharmaceutically acceptable salt thereof.

8. A pharmaceutical preparation which comprises a quantity, which is jointly effective for treating a proliferative disease that can be treated by administration of an abl-, PDGF-R-(platelet-derived growth factor receptor) and/or Kit receptor-tyrosine kinase inhibitor, of a combination according to claim 2, wherein any component (a) and/or (b) can also be present in the form of a pharmaceutically acceptable salt, if at least one salt-forming group is present, with one or more pharmaceutically acceptable carrier materials.

## Patentansprüche

1. Verwendung einer Kombination von
(a) wenigstens einem abl-, PDGF-R- (Plättchen-abgeleiteter Wachstumsfaktorrezeptor) und/oder Kit-Rezeptor-Tyrosinkinase-Inhibitor und
(b) wenigstens einer organischen Verbindung, die zur Bindung an ein saures α₁-Glycoprotein fähig ist,
worin die Komponenten (a) und/oder (b) auch in der Form eines pharmazeutisch akzeptablen Salzes vorliegen können, sofern wenigstens eine ein Salz bildende Gruppe vorhanden ist, zur Herstellung eines pharmazeutischen Präparats für eine Verwendung als Zusammensetzungen gegen eine proliferative Krankheit, die behandelt werden kann durch Verabreichung eines abl-, PDGF-R- und/oder Kit-Rezeptor-Tyrosinkinase-Inhibitors, der gegenüber einer solchen Behandlung resistent ist oder während einer Behandlung mit 4-(4-Methylpiperazin-1-ylmethyl)-N-[4-methyl-3-(4-pyridin-3-yl)-pyrimidin-2-ylamino)-phenyl]-benzamid resistent wird oder vollständig oder teilweise resistent geworden ist.

2. Kombinationspräparat, umfassend (a) wenigstens einen abl-, PDGF-R- (Plättchen-abgeleiteter Wachstumsfaktorrezeptor) und/oder Kit-Rezeptor-Tyrosinkinase-Inhibitor und (b) wenigstens eine organische Verbindung, die zur Bindung an ein saures α₁-Glycoprotein (AGP) fähig ist, oder pharmazeutisch akzeptable Salze der Komponenten (a), (b) oder (a) und (b), sofern wenigstens eine ein Salz bildende Gruppe vorhanden ist, und einen pharmazeutisch akzeptablen Träger, worin der abl-, PDGF-Rezeptor-und/oder Kit-Rezeptor-Tyrosinkinase-Inhibitor (a) ausgewählt ist aus
(i) 1-(4-Chloranilino)-4-(4-pyridylmethyl)-phthalazin und
(ii) einem N-Phenyl-2-pyrimidinaminderivat der Formel I 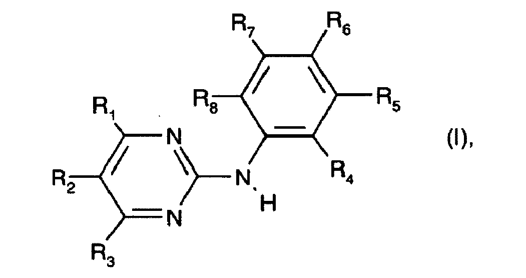
worin
R₁ für Pyrazinyl, 1-Methyl-1 H-pyrrolyl, oder Amino- oder Aminoniederalkyl-substituiertes Phenyl steht, worin die Aminogruppe jeweils frei, alkyliert oder acyliert ist, 1H-Indolyl oder 1H-Imidazolyl, das an ein 5-gliedriges Ringkohlenstoffatom gebunden ist, oder unsubstituiertes oder Niederalkyl-substituiertes Pyridyl, das an ein Ringkohlenstoffatom gebunden ist und unsubstituiert oder am Stickstoffatom durch Sauerstoff substituiert ist,
R₂ und R₃ jeweils unabhängig voneinander für Wasserstoff oder Niederalkyl stehen, und
ein oder zwei der Reste R₄, R₅, R₆, R₇ und R₈ jeweils für Nitro, durch Fluor substituiertes Niederalkoxy oder einen Rest der folgenden Formel II stehen
-N(R₉)-C(=X)-(Y)ₙ-R₁₀) (II),
worin
R₉ für Wasserstoff oder Niederalkyl steht,
X für Oxo, Thio, Imino, N-Niederalkylimino, Hydroxyimino oder O-Niederalkylhydroxyimino steht,
Y für Sauerstoff oder die Gruppe NH steht,
n für 0 oder 1 steht, und
R₁₀ für einen aliphatischen Rest mit wenigstens 5 Kohlenstoffatomen oder einen aromatischen, aromatisch-aliphatischen, cycloaliphatischen, cylcoaliphatisch-aliphatischen, heterocyclischen oder heterocyclisch-aliphatischen Rest steht, und die verbleibenden Reste R₄, R₅, R₆, R₇ und R₈ jeweils unabhängig voneinander für Wasserstoff, Niederalkyl, das unsubstituiert oder substituiert ist durch freies oder alkyliertes Amino, Piperazinyl, Piperidinyl, Pyrrolidinyl oder Morpholinyl, oder für Niederalkanoyl, Trifluormethyl, freies, verethertes oder verestertes Hydroxy, freies, alkyliertes oder acyliertes Amino oder freies oder verestertes Carboxy steht, oder
ein Salz solcher Verbindungen mit wenigstens einer ein Salz bildenden Gruppe, und
die organische Gruppe (b), welche zur Bindung an ein saures α₁-Glycoprotein (AGP) befähigt ist, ausgewählt ist aus:
Nicergolin, Prazosin, Alfentanil, Ketamin, Ethidocain, Fentanil, Meperidin, Methadon, Phenylbutazon, Bupivacain, Etidocain, Phencyclidin, Lidocain, Phencyclidin, Aprindin, Disopyramid, Chinidin, Verapamil, Erythromycin, Acenocumarol, Dipyridamol, Ticlopidin, Warfarin, Phenytoin, Carbamazepin, Naproxen, Alprenolol, Metoprolol, Oxprenolol, Pindolol, Propranolol, Timolol, Progesteron, Cortexon, Testosteron, Estradiol, Prednisolon, Metocurin, d-Tubocurarin, Amitriptylin, Chlorpromazin, Cyclazindol, Desmethylimipramin, Diazepam, Doxepin, Flurazepam, Fluphenazin, Haloperidol, Imipramin, Loxapin, Mianserin, Nortriptylin, Norzimelidin, Perazin, Perphenazin, Phenobarbital, Phenothiazinderivaten, Promazin, Acepromazin, Protipendyl, Thioridazin, Thiothixen, Triazolam, Trifluoperazin, Zimelidin, Vitamin B₁₂, Folsäure, 1,8-Anilinonaphthalinsulfonat, Aminopyrin, Amoxapin, Bupropion, Maprotilin, Nomifensin, Trazodon, Ritodrin, Doxazosin, Trimazosin, Binedalin, Amsacrin, Apazon, Ciclazindol, Indomethacin, Probenecid, Retinolsäure, Sulfinpyrazon, Tolmetin, Benoxaprofen, Heparin, Sufentanil, Lofentanil, Metoclopramid, Nicardipin, Pirmenol, Mifepriston, Aprindil, Auramin O, Bepridil, Desipramin, Desmethylclomipramin, Moxaprindin, Chinin, Lorcainid, Prothipendyl, Protriptylin, Trihexyphenidyl, Biperiden, Methaqualon, Diphenhydramin, Glutethimid, Chlordiazepoxid, L-Tryptophan, Mepivacain, Levomethadon, Opipramol, Trifluopromazin, Trimipramin, Trisbutoxyethylphosphat, Staurosporin, N-Benzoylstaurosporin und 7-Hydroxystaurosporin,
worin die Komponenten (a) und/oder (b) auch in der Form eines pharmazeutisch akzeptablen Salzes vorliegen können, falls wenigstens eine ein Salz bildende Gruppe vorhanden ist.

3. Kombinationspräparat nach Anspruch 2, worin
(a) wenigstens ein abl-, PDGF-R- und/oder Kit-Rezeptor-Tyrosinkinase-Inhibitor, der ausgewählt ist aus der Gruppe, die besteht aus 1-(4-Chloranilino)-4-(4-pyridylmethyl)-phthalazin und 4-(4-Methylpiperazin-1-ylmethyl)-N-[4-methyl-3-(4-pyridin-3-yl)-pyrimidin-2-ylamino)-phenyl]-benzamid, oder einem pharmazeutisch akzeptablen Salz von ein oder mehr dieser Verbindungen, und
(b) wenigstens eine Verbindung, die zur Bindung an ein saures α₁-Glycoprotein fähig ist, die ausgewählt ist aus der Gruppe, die besteht aus einem Antibiotikum, Staurosporin, N-Benzoylstaurosporin und 7-Hydroxystaurosporin, oder einem pharmazeutisch akzeptablen Salz hiervon, kombiniert sind.

4. Kombinationspräparat nach Anspruch 2, worin (a) der abl-, PDGF-Rezeptor- und/oder Kit-Rezeptor-Tyrosinkinase-Inhibitor 4-(4-Methylpiperazin-1-ylmethyl)-N-[4-methyl-3-(4-pyridin-3-yl)-pyrimidin-2-yl-amino)-phenyl]-benzamid oder ein pharmazeutisch akzeptables Salz hiervon ist, und (b) die zur Bindung an ein saures α₁-Glycoprotein befähigte Verbindung Erythromycin ist, oder ein pharmazeutisch akzeptables Salz hiervon.

5. Produkt, umfassend
(a) wenigstens ein abl-, PDGF-R- (Plättchen-abgeleiteter Wachstumsfaktorrezeptor) und/oder Kit-Rezeptor-Tyrosinkinase-Inhibitor, und
(b) wenigstens eine Verbindung, die zur Bindung an ein saures α₁-Glycoprotein fähig ist,
worin die Komponenten (a) und/oder (b) auch in der Form eines pharmazeutisch akzeptablen Salzes vorliegen können, falls wenigstens eine ein Salz bildende Gruppe vorhanden ist,
in der Gegenwart oder Abwesenheit von ein oder mehr pharmazeutisch akzeptablen Trägermaterialien, als ein Kombinationspräparat für eine simultane oder chronologisch gestaffelte Anwendung innerhalb einer Zeitdauer, die ausreichend kurz ist für die Wirkstoffe sowohl der Komponente (a) als auch der Komponente (b), um eine antiproliferative Wirksamkeit der Verbindung (a) gegen proliferative Zellen, speziell in einem Patienten, zu verbessern, für die Behandlung einer proliferativen Krankheit, die auf eine solche Verbindung antwortet, worin der abl-, PDGF-Rezeptor- und/oder Kit-Rezeptor-Tyrosinkinase-Inhibitor ausgewählt ist aus der Gruppe, die besteht aus
(i) 1-(4-Chloranilino)-4-(4-pyridylmethyl)-phthalazin und
(ii) einem N-Phenyl-2-pyrimidinaminderivat der Formel I 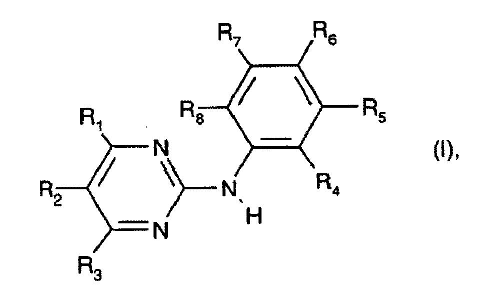
worin
R₁ steht für Pyrazinyl, 1-Methyl-1H-pyrrolyl, oder Amino- oder Aminoniederalkyl-substituiertes Phenyl steht, worin die Aminogruppe jeweils frei, alkyliert oder acyliert ist, 1H-Indolyl oder 1H-Imidazolyl, das an ein 5-gliedriges Ringkohlenstoffatom gebunden ist, oder unsubstituiertes oder Niederalkyl-substituiertes Pyridyl, das an ein Ringkohlenstoffatom gebunden ist und unsubstituiert oder am Stickstoffatom durch Sauerstoff substituiert ist,
R₂ und R₃ jeweils unabhängig voneinander für Wasserstoff oder Niederalkyl stehen, und
ein oder zwei der Reste R₄, R₅, R₆, R₇ und R₈ jeweils für Nitro, durch Fluor substituiertes Niederalkoxy oder einen Rest der folgenden Formel II stehen,
-N(R₉)-C(=X)-(Y)ₙ-R₁₀ (II),
worin
R₉ für Wasserstoff oder Niederalkyl steht,
X für Oxo, Thio, Imino, N-Niederalkylimino, Hydroxyimino oder O-Niederalkylhydroxyimino steht,
Y für Sauerstoff oder die Gruppe NH steht,
n für 0 oder 1 steht, und
R₁₀ für einen aliphatischen Rest mit wenigstens 5 Kohlenstoffatomen oder einen aromatischen, aromatisch-aliphatischen, cycloaliphatischen, cylcoaliphatisch-aliphatischen, heterocyclischen oder heterocyclisch-aliphatischen Rest steht, und die verbleibenden Reste R₄, R₅, R₆, R₇ und R₈ jeweils unabhängig voneinander für Wasserstoff, Niederalkyl, das unsubstituiert oder substituiert ist durch freies oder alkyliertes Amino, Piperazinyl, Piperidinyl, Pyrrolidinyl oder Morpholinyl, oder für Niederalkanoyl, Trifluormethyl, freies, verethertes oder verestertes Hydroxy, freies, alkyliertes oder acyliertes Amino oder freies oder verestertes Carboxy steht, oder
ein Salz solcher Verbindungen mit wenigstens einer ein Salz bildenden Gruppe, und
die organische Gruppe (b), welche zur Bindung an ein saures α₁-Glycoprotein (AGP) befähigt ist, ausgewählt ist aus:
Nicergolin, Prazosin, Alfentanil, Ketamin, Ethidocain, Fentanil, Meperidin, Methadon, Phenylbutazon, Bupivacain, Etidocain, Phencyclidin, Lidocain, Phencyclidin, Aprindin, Disopyramid, Chinidin, Verapamil, Erythromycin, Acenocumarol, Dipyridamol, Ticlopidin, Warfarin, Phenytoin, Carbamazepin, Naproxen, Alprenolol, Metoprolol, Oxprenolol, Pindolol, Propranolol, Timolol, Progesteron, Cortexon, Testosteron, Estradiol, Prednisolon, Metocurin, d-Tubocurarin, Amitriptylin, Chlorpromazin, Cyclazindol, Desmethylimipramin, Diazepam, Doxepin, Flurazepam, Fluphenazin, Haloperidol, Imipramin, Loxapin, Mianserin, Nortriptylin, Norzimelidin, Perazin, Perphenazin, Phenobarbital, Phenothiazinderivaten, Promazin, Acepromazin, Protipendyl, Thioridazin, Thiothixen, Triazolam, Trifluoperazin, Zimelidin, Vitamin B₁₂, Folsäure, 1,8-Anilinonaphthalinsulfonat, Aminopyrin, Amoxapin, Bupropion, Maprotilin, Nomifensin, Trazodon, Ritodrin, Doxazosin, Trimazosin, Binedalin, Amsacrin, Apazon, Ciclazindol, Indomethacin, Probenecid, Retinolsäure, Sulfinpyrazon, Tolmetin, Benoxaprofen, Heparin, Sufentanil, Lofentanil, Metoclopramid, Nicardipin, Pirmenol, Mifepriston, Aprindil, Auramin O, Bepridil, Desipramin, Desmethylclomipramin, Moxaprindin, Chinin, Lorcainid, Prothipendyl, Protriptylin, Trihexyphenidyl, Biperiden, Methaqualon, Diphenhydramin, Glutethimid, Chlordiazepoxid, L-Tryptophan, Mepivacain, Levomethadon, Opipramol, Trifluopromazin, Trimipramin, Trisbutoxyethylphosphat, Staurosporin, N-Benzoylstaurosporin und 7-Hydroxystaurosporin,
worin die Komponenten (a) und/oder (b) auch in der Form eines pharmazeutisch akzeptablen Salze vorliegen können, falls wenigstens eine ein Salz bildende Gruppe vorhanden ist.

6. Produkt nach Anspruch 5, worin (a) der ein abl-, PDGF-R- und/oder Kit-Rezeptor-Tyrosinkinase-Inhibitor ausgewählt ist aus 1-(4-Chloranilino)-4-(4-pyridylmethyl)-phthalazin und 4-(4-Methylpiperazin-1-ylmethyl)-N-[4-methyl-3-(4-pyridin-3-yl)-pyrimidin-2-ylamino)-phenyl]-benzamid, oder einem pharmazeutisch akzeptablen Salz von ein oder mehr dieser Verbindungen, und
(b) die zur Bindung an ein saures α₁-Glycoprotein fähige Verbindung ausgewählt ist aus einem Antibiotikum, Staurosporin, N-Benzoylstaurosporin und 7-Hydroxystaurosporin, oder einem pharmazeutisch akzeptablen Salz einer oder mehrerer dieser Verbindungen.

7. Produkt nach Anspruch 5, worin (a) der abl-, PDGF-Rezeptor- und/oder Kit-Rezeptor-Tyrosinkinase-Inhibitor 4-(4-Methylpiperazin-1-ylmethyl)-N-[4-methyl-3-(4-pyridin-3-yl)-pyrimidin-2-ylamino)-phenyl]-benzamid oder ein pharmazeutisch akzeptables Salz hiervon ist, und (b) die zur Bindung an ein saures α₁-Glycoprotein befähigte Verbindung Erythromycin ist, oder ein pharmazeutisch akzeptables Salz hiervon.

8. Pharmazeutisches Präparat, umfassend eine Menge einer Kombination nach Anspruch 2, die gemeinsam für eine Behandlung einer proliferativen Krankheit wirksam ist, welche durch Verabreichung eines abl-, PDGF-R- (Plättchen-abgeleiteter Wachtsumsfaktorrezeptor) und/oder Kit-Rezeptor-Tyrosinkinase-Inhibitors behandelt werden kann, worin die Komponenten (a) und/oder (b) auch in der Form eines pharmazeutisch akzeptablen Salzes vorliegen können, falls wenigstens eine ein Salz bildende Gruppe vorhanden ist, mit ein oder mehr pharmazeutisch akzeptablen Trägermaterialien.

## Revendications

1. Utilisation d'une combinaison de
(a) au moins un inhibiteur de abl-, PDGF-R- (récepteur du facteur de croissance dérivé des plaquettes) et/ou récepteur Kit-tyrosine kinase et
(b) au moins un composé organique capable de se lier à une glycoprotéine α₁-acide,
où n'importe quels composants (a) et/ou (b) peuvent être également présents sous la forme d'un sel pharmaceutiquement acceptable, si au moins un groupe formant un sel est présent,
pour produire une préparation pharmaceutique destinée à être utilisée comme compositions contre une maladie proliférante que l'on peut traiter par administration d'un inhibiteur de abl-, PDGF-R- et/ou récepteur Kit-tyrosine kinase qui, lors d'un traitement au 4-(4-méthylpipérazin-1-ylméthyl)-N-[4-méthyl-3-(4-pyridin-3-yl)pyrimidin-2-ylamino)phényl]-benzamide, devient ou est devenu complètement ou partiellement résistante à un tel traitement.

2. Préparation de combinaison comprenant (a) au moins un inhibiteur de abl-, PDGF-R- (récepteur du facteur de croissance dérivé des plaquettes) et/ou récepteur Kit- tyrosine kinase et (b) au moins un composé organique capable de se lier à une glycoprotéine α₁-acide (AGP); ou les sels pharmaceutiquement acceptables de n'importe quels composants (a), (b) ou (a) et (b) si au moins un groupe formant un sel est présent, et un véhicule pharmaceutiquement acceptable, où l'inhibiteur de abl-, Récepteur de PDGF- et/ou récepteur Kit- tyrosine kinase (a) est choisi parmi:
(i) la 1-(4-chloro-anilino)-4-(4-pyridyl-méthyl)phtalazine
et
(ii) un dérivé de N-phényl-2-pyrimidine-amine de formule I 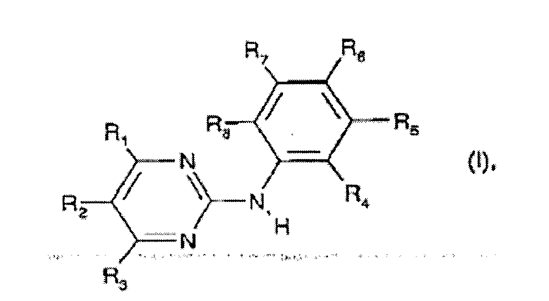
dans laquelle
R₁ est un groupe pyrazinyle, 1-méthyl-1H-pyrrolyle, phényle substitué par un groupe amino ou amino-alkyle inférieur, où dans chaque cas, le groupe amino est libre, alkylé ou acylé, 1H-indolyle ou 1H-imidazolyle lié à un atome de carbone de cycle à cinq chaînons, ou pyridyle non substitué ou substitué par un radical alkyle inférieur, lié à un atome de carbone de cycle et non substitué ou substitué au niveau de l'atome d'azote par un oxygène,
R₂ et R₃ représentent chacun indépendamment l'un de l'autre un atome d'hydrogène ou un radical alkyle inférieur,
un ou deux des radicaux R₄, R₅, R₆, R₇ et R₈ représentent chacun un groupe nitro, alcoxy inférieur fluoré ou un radical de formule II
-N(R₉)-C(-X)-(Y)ₙ-R₁₀ (II)
dans laquelle
R₉ est un atome d'hydrogène ou un radical alkyle inférieur,
X représente un groupe oxo, thio, imino, N-alkyl(inférieur)-imino, hydroxyimino ou O-alkyl(inférieur)-hydroxyimino,
Y représente un atome d'oxygène ou le groupe NH,
n est égal à 0 ou 1 et
R₁₀ est un radical aliphatique ayant au moins 5 atomes de carbone, ou un radical aromatique, aromatique-aliphatique, cycloaliphatique, cycloaliphatique-aliphatique, hétérocyclique ou hétérocyclique-aliphatique, et les radicaux R₄, R₅, R₆, R₇ et R₈ restants représentent chacun indépendamment les uns des autres un atome d'hydrogène, un radical alkyle inférieur qui est non substitué ou substitué par un groupe amino libre ou alkylé, pipérazinyle, pipéridinyle, pyrrolidinyle ou par un groupe morpholinyle, ou alcanoyle inférieur, trifluorométhyle, hydroxy libre, éthérifié ou estérifié, amino libre, alkylé ou acylé ou carboxy libre ou estérifié, ou
un sel de tels composés ayant au moins un groupe formant un sel;
et le composé organique capable de se lier à la glycoprotéine α₁-acide (AGP) (b) est choisi parmi:
Nicergoline, Prazosine, Alfentanil, Kétamine, Ethidocaïne, Fentanil, Mépéridine, Méthadone, Phénylbutazone, Bupivacaïne, Etidocaïne, Phencyclidine, Lidocaïne, Phencyclidine, Aprindine, Disopyramide, Quinidine, Vérapamil, Erythromycine, Acénocoumarol, Dipyridamole, Ticlopidine, Warfarine, Phénytoïne, Carbamazépine, Naproxène, Alprénolol, Métoprolol, Oxprénolol, Pindolol, Propranolol, Timolol, Progestérone, Cortexone, Testostérone, Estradiol, Prednisolone, Métocurine, d-Tubocurarine, Amitriptyline, Chlorpromazine, Cyclazindol, Desméthylimipramine, Diazépam, Doxépine, Flurazépam, Fluphénazine, Halopéridol, Imipramine, Loxapine, Miansérine, Nortriptyline, Norzimélidine, Perazine, Perphénazine, Phénobarbital, dérivés de Phénothiazine, Promazine, Acépromazine, Protipendyle, Thioridazine, Thiothixène, Triazolam, Trifluoperazine, Zimélidine, Vitamine B₁₂, Acide folique, sulfonate de 1,8-Anilino-naphtalène, Aminopyrine, Amoxapine, Bupropion, Maprotiline, Nomifensine, Trazodone, Ritodrine, Doxazosine, Trimazosine, Binédaline, Amsacrine, Apazone, Ciclazindol, Indométhacine, Probénécide, Acide rétinoïque, Sulfinpyrazone, Tolmétine, Bénoxaprofène, Héparine, Sufentanil, Lofentanil, Métoclopramide, Nicardipine, Pirménol, mifépristone, Aprindil, Auramine O, Bépridil, Désipramine, Desméthylclomipramine, Moxaprindine, Quinine, Lorcaïnide, Prothipendyl, Protriptyline, Trihexyphénidyle, Bipéridène, Méthaqualone, Diphénhydramine, Glutéthimide, Chlordiazépoxyde, L-Tryptophane, Mépivacaïne, Lévométhadone, Opipramol, Trifluopromazine, Trimipramine, phosphate de tris-butoxyéthyle, staurosporine, N-benzoyl-staurosporine et 7-hydroxystaurosporine;
dans laquelle n'importe quels composants (a) et/ou (b) peuvent être également présents sous la forme d'un sel pharmaceutiquement acceptable, si au moins un groupe formant un sel est présent.

3. Préparation de combinaison selon la revendication 2, dans laquelle (a) au moins un inhibiteur de abl-, Récepteur de PDGF- et/ou récepteur Kit- tyrosine kinase choisi dans le groupe constitué par la 1-(4-chloro-anilino)-4-(4-pyridyl-méthyl)phtalazine et le 4-(4-méthylpipérazin-1-ylméthyl)-N-[4-méthyl-3-(4-pyridin-3-yl)pyrimidin-2-ylamino)phényl]benzamide, ou un sel pharmaceutiquement acceptable de l'un quelconque de ces composés ou de plusieurs d'entre eux; et
(b) au moins un composé capable de se lier à une glycoprotéine α₁-acide choisi dans le groupe constitué par un antibiotique, la staurosporine, la N-benzoyl-staurosporine et la 7-hydroxy-staurosporine, ou l'un de leurs sels pharmaceutiquement acceptables, sont combinés.

4. Préparation de combinaison selon la revendication 2, dans laquelle (a) l'inhibiteur de abl-, Récepteur de PDGF- et/ou récepteur Kit-tyrosine kinase est le 4-(4-méthylpipérazin-1-ylméthyl)-N-[4-méthyl-3-(4-pyridin-3-yl)pyrirnidin-2-ylamino)phényl]benzamide, ou un de ses sels pharmaceutiquement acceptables, et (b) le composé capable de se lier à une glycoprotéine α₁-acide est l'Erythromycine, ou l'un de ses sels pharmaceutiquement acceptables.

5. Produit qui comprend
(a) au moins un inhibiteur de abl-, PDGF-R- (récepteur du facteur de croissance dérivé des plaquettes) et/ou récepteur Kit- tyrosine kinase et
(b) au moins un composé organique capable de se lier à une glycoprotéine α₁-acide,
où n'importe quels composants (a) et/ou (b) peuvent être également présents sous la forme d'un sel pharmaceutiquement acceptable, si au moins un groupe formant un sel est présent,
en présence ou en l'absence d'une ou de plusieurs substances véhicules pharmaceutiquement acceptables, sous forme d'une préparation de combinaison pour une utilisation simultanée ou chronologiquement échelonnée en une période de temps qui est suffisamment courte pour que les composés actifs aussi bien du composant (a) que du composant (b) renforcent l'activité antiproliférante du composé (a) contre les cellules proliférantes, en particulier chez un patient, pour traiter une maladie proliférante qui répond à un tel composé, dans lequel l'inhibiteur de abl-, Récepteur de PDGF- et/ou récepteur Kit- tyrosine kinase est choisi dans le groupe constitué par
(i) la 1-(4-chloro-anilino)-4-(4-pyridyl-méthyl)phtalazine et
(ii) un dérivé de N-phényl-2-pyrimidine-amine de formule I 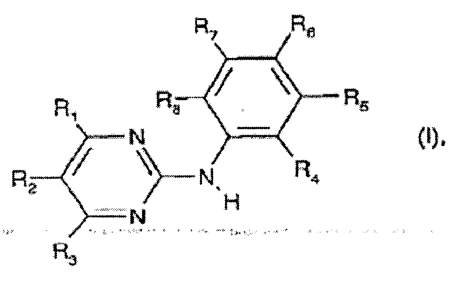
dans laquelle
R₁ est un groupe pyrazinyle, 1-méthyl-1H-pyrrolyle, phényle substitué par un groupe amino ou amino-alkyle inférieur, où dans chaque cas, le groupe amino est libre, alkylé ou acylé, 1H-indolyle ou 1H-imidazolyle lié à un atome de carbone de cycle à cinq chaînons, ou pyridyle non substitué ou substitué par un radical alkyle inférieur, lié à un atome de carbone de cycle et non substitué ou substitué au niveau de l'atome d'azote par un oxygène,
R₂ et R₃ représentent chacun indépendamment l'un de l'autre un atome d'hydrogène ou un radical alkyle inférieur,
un ou deux des radicaux R₄, R₅, R₆, R₇ et R₈ représentent chacun un groupe nitro, alcoxy inférieur fluoré ou un radical de formule II
-N(R₉)-C(-X)-(Y)ₙ-R₁₀ (II)
dans laquelle
R₉ est un atome d'hydrogène ou un radical alkyle inférieur,
X représente un groupe oxo, thio, imino, N-alkyl(inférieur)-imino, hydroxyimino ou O-alkyl(inférieur)-hydroxyimino,
Y représente un atome d'oxygène ou le groupe NH,
n est égal à 0 ou 1 et
R₁₀ est un radical aliphatique ayant au moins 5 atomes de carbone, ou un radical aromatique, aromatique-aliphatique, cycloaliphatique, cycloaliphatique-aliphatique, hétérocyclique ou hétérocyclique-aliphatique, et les radicaux R₄, R₅, R₆, R₇ et R₈ restants représentent chacun indépendamment les uns des autres un atome d'hydrogène, un radical alkyle inférieur qui est non substitué ou substitué par un groupe amino libre ou alkylé, pipérazinyle, pipéridinyle, pyrrolidinyle ou par un groupe morpholinyle, ou alcanoyle inférieur, trifluorométhyle, hydroxy libre, éthérifié ou estérifié, amino libre, alkylé ou acylé ou carboxy libre ou estérifié, ou
un sel de tels composés ayant au moins un groupe formant un sel;
et le composé organique capable de se lier à la glycoprotéine α₁-acide (AGP) (b) est choisi parmi:
Nicergoline, Prazosine, Alfentanil, Kétamine, Ethidocaïne, Fentanil, Mépéridine, Méthadone, Phénylbutazone, Bupivacaïne, Etidocaïne, Phencyclidine, Lidocaïne, Phencyclidine, Aprindine, Disopyramide, Quinidine, Vérapamil, Erythromycine, Acénocoumarol, Dipyridamole, Ticlopidine, Warfarine, Phénytoïne, Carbamazépine, Naproxène, Alprénolol, Métoprolol, Oxprénolol, Pindolol, Propranolol, Timolol, Progestérone, Cortexone, Testostérone, Estradiol, Prednisolone, Métocurine, d-Tubocurarine, Amitriptyline, Chlorpromazine, Cyclazindol, Desméthylimipramine, Diazépam, Doxépine, Flurazépam, Fluphénazine, Halopéridol, Imipramine, Loxapine, Miansérine, Nortriptyline, Norzimélidine, Perazine, Perphénazine, Phénobarbital, dérivés de Phénothiazine, Promazine, Acépromazine, Protipendyle, Thioridazine, Thiothixène, Triazolam, Trifluoperazine, Zimélidine, Vitamine B₁₂, Acide folique, sulfonate de 1,8-Anilino-naphtalène, Aminopyrine, Amoxapine, Bupropion, Maprotiline, Nomifensine, Trazodone, Ritodrine, Doxazosine, Trimazosine, Binédaline, Amsacrine, Apazone, Ciclazindol, Indométhacine, Probénécide, Acide rétinoïque, Sulfinpyrazone, Tolmétine, Bénoxaprofène, Héparine, Sufentanil, Lofentanil, Métoclopramide, Nicardipine, Pirménol, mifépristone, Aprindil, Auramine O, Bépridil, Désipramine, Desméthylclomipramine, Moxaprindine, Quinine, Lorcaïnide, Prothipendyl, Protriptyline, Trihexyphénidyle, Bipéridène, Méthaqualone, Diphénhydramine, Glutéthimide, Chlordiazépoxyde, L-Tryptophane, Mépivacaïne, Lévométhadone, Opipramol, Trifluopromazine, Trimipramine, phosphate de tris-butoxyéthyle, staurosporine, N-benzoyl-staurosporine et 7-hydroxystaurosporine;
dans lequel n'importe quels composants (a) et/ou (b) peuvent être également présents sous la forme d'un sel pharmaceutiquement acceptable, si au moins un groupe formant un sel est présent.

6. Produit selon la revendication 5, dans lequel (a) l'inhibiteur de abl-, Récepteur de PDGF- et/ou récepteur Kit- tyrosine kinase est choisi parmi la 1-(4-chloro-anilino)-4-(4-pyridyl-méthyl)phtalazine et le 4-(4-méthylpipérazin-1-ylméthyl)-N-[4-méthyl-3-(4-pyridin-3-yl)pyrimidin-2-ylamino)phényl]benzamide, ou un sel pharmaceutiquement acceptable de l'un quelconque de ces composés ou de plusieurs d'entre eux; et
(b) le composé organique capable de se lier à une glycoprotéine α₁-acide choisi parmi un antibiotique, la staurosporine, la N-benzoyl-staurosporine et la 7-hydroxy-staurosporine, ou un sel pharmaceutiquement acceptable de l'un quelconque de ces composés ou de plusieurs d'entre eux.

7. Produit selon la revendication 5, dans lequel (a) l'inhibiteur de abl-, Récepteur de PDGF- et/ou récepteur Kit- tyrosine kinase est le 4-(4-méthylpipérazin-1-ylméthyl)-N-[4-méthyl-3-(4-pyridin-3-yl)pyrimidin-2-ylamino)phényl]benzamide, ou un de ses sels pharmaceutiquement acceptables, et (b) le composé capable de se lier à une glycoprotéine α₁-acide est l'Erythromycine, ou l'un de ses sels pharmaceutiquement acceptables.

8. Préparation pharmaceutique qui comprend une quantité, qui est conjointement efficace pour le traitement d'une maladie proliférante que l'on peut traiter par administration d'un inhibiteur de abl-, PDGF-R-(récepteur du facteur de croissance dérivé des plaquettes) et/ou récepteur Kit- tyrosine kinase, d'une combinaison selon la revendication 2, dans laquelle n'importe quels composants (a) et/ou (b) peuvent être également présents sous la forme d'un sel pharmaceutiquement acceptable, si au moins un groupe formant un sel est présent, avec une ou plusieurs substances véhicules pharmaceutiquement acceptables.
